(19) Europäisches Patentamt
European Patent Office
Office européen des brevets

(11) EP 2 982 698 A1

(12) **EUROPEAN PATENT APPLICATION**

(43) Date of publication:
**10.02.2016 Bulletin 2016/06**

(21) Application number: **14180423.7**

(22) Date of filing: **08.08.2014**

(51) Int Cl.:
***C07K 17/08*** *(2006.01)* ***C07K 17/12*** *(2006.01)*
***C12N 11/08*** *(2006.01)* ***C12N 11/12*** *(2006.01)*
***G01N 33/544*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Commissariat à l'Énergie Atomique et aux Énergies Alternatives**
**75015 Paris (FR)**

(72) Inventors:
- **Credou, Julie**
  **94230 Cachan (FR)**
- **Berthelot, Thomas**
  **91940 Les Ulis (FR)**
- **Faddoul, Rita**
  **91300 Massy (FR)**

(74) Representative: **Icosa**
**83 avenue Denfert-Rochereau**
**75014 Paris (FR)**

(54) **Method for photo-immobilizing biomolecules on substantially non-porous carriers**

(57) The present invention relates to the immobilization of biomolecules on substantially non-porous carriers by irradiating the biomolecule-impregnated carriers with a light of a wavelength of at least 340 nm.

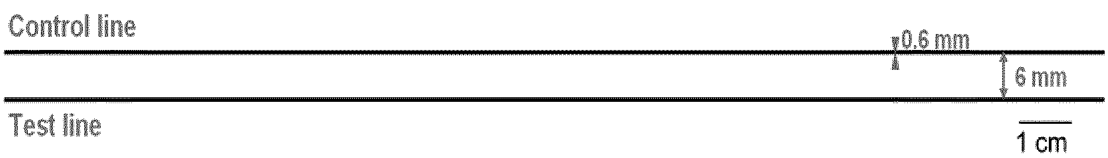


**FIG. 1**

EP 2 982 698 A1

## Description

### FIELD OF INVENTION

The present invention relates to the immobilization of biomolecules on substantially non-porous carriers by irradiating the biomolecule-impregnated carriers with a light of a wavelength of at least 340 nm.

### BACKGROUND OF INVENTION

Though paper-based immunoassay such as dipstick tests or lateral flow immunoassays (LFIAs) have been marketed and extensively employed for point-of-care (POC) diagnostics and pathogen detection since the 80s (diabetes and pregnancy tests being the most famous), the recent impetus given to paper-based microfluidics by American, Canadian and Finnish research teams has resulted in the development of new paper-based bioanalytical devices with complex designs allowing multiplex diagnosis.

Equipped with this sustainable, low-cost and easy to use material, the next challenge now lies in the production process. Two parts of the process should therefore be considered: the whole device design and shaping on one hand, and the biosensing material dispensing and immobilization on the other hand.

Regarding the device shaping, the frame material of a multiplex device needs to be patterned with microfluidic channels. Thus, several methods for patterning paper sheets have been developed. Among the many processes are photolithography, using SU-8 or SC photoresist, "wax printing" or "wax dipping", inkjet printing and laser cutting.

With regard to the biosensing material, the spatially controlled immobilization of biomolecules is a key step in the development of biosensing devices. Photolithographic methods can be used to control protein immobilization onto selected specific areas of the substrate. Yet, this is a long and complex process composed of many steps. First of all, a photoresist is prepared and deposited onto the substrate through a master form. After UV exposure, the non-exposed regions are developed by chemical treatment. Biomolecules are finally immobilized on the non-developed regions. In addition to complexity, and resulting high cost, there is a not insignificant risk for biomolecules to come across traces of the toxic reagents and solvents used in the development step.

This is why printing techniques such as micro-contact printing or inkjet printing are often preferred to spatially control biomolecule immobilization. Compared to photolithography, printing techniques allow quick cycles where only one step - printing biomolecule - is required. Moreover, printing is considered a biocompatible environmentally friendly process. It is a versatile technique enabling the deposition of variable kinds of solutions (biomolecules, polymers, solvents, metals) onto different types of substrates (cellulose, polymer, glass, silicon) and according to any design desired. It is a fast dispensing process allowing low-cost, high throughput fabrication, and therefore a very attractive approach regarding the economic and ecological goals. However, to be able to detect an immune answer, many printing cycles were needed so far. For example, 60 print cycles of an immune-sensing ink were necessary to detect 10 $\mu g\ L^{-1}$ (*i.e.* 10 $ng\ mL^{-1}$) of IgG molecule (Abe et al., Anal. Bioanal. Chem., 2010, 398, 885-893) and 24 cycles of protein ink were inkjet printed in order to detect 0.8 $\mu M$ of human serum albumin (HSA) (*i.e.* 53.6 $\mu g\ mL^{-1}$ since $M_{HSA}$ = 67 kDa) (Abe et al., Anal. Chem. 2008, 80, 6928-6934). Moreover, printing is only a dispensing technique and is not sufficient by itself to strongly immobilize biomolecules onto cellulose. Recent findings revealed that about 40% of antibody molecules adsorbed onto cellulose paper can actually desorb from the fibers (Jarujamrus et al., Analyst 2012, 137, 2205-2210). Direct adsorption of antibodies onto cellulose is therefore too weak to allow the permanent immobilization required in the development on immunoassay and cellulose activation or functionalization is thus necessary.

The present inventors have surprisingly discovered a method allowing biomolecules, including in particular antibodies, to be strongly immobilized on a wide range of naturally non-porous materials as well as on pretreated porous materials (e.g. cellulose), which allows the biological activity of the biomolecules to be preserved. The method of the invention is in particular advantageously compatible with inkjet printing techniques as well as with the above discussed economic and ecological goals, since cellulose can also be used as a carrier.

### SUMMARY

This invention thus relates to a process for immobilizing biomolecules on a substantially non-porous carrier comprising the steps of:

(i) impregnating said carrier with a solution containing the said biomolecules; and
(ii) irradiating the impregnated carrier resulting from step (i) with a light of a wavelength of at least 340 nm;

wherein said carrier is composed of a naturally substantially non-porous material, or of a porous material that has been rendered substantially non-porous; and wherein said biomolecules are not functionalized.

In a particular embodiment, the process of the invention further comprises a step of drying said impregnated carrier after step (i) and before step (ii).

In a particular embodiment, the process of the invention further comprises at least one step of washing the irradiated carrier resulting from step (ii).

In a particular embodiment, the irradiation light has a wavelength of from 340 nm to 800 nm, preferably of from 340 nm to 400 nm, preferably of from 400 nm to 800 nm, and is preferably of about 365 nm.

In a particular embodiment, the irradiation step (ii) is performed with a photoenergy of from 1 $mJ/cm^2$ to 500 $J/cm^2$, preferably of from 1 $J/cm^2$ to 80 $J/cm^2$, and is preferably of about 10 $J/cm^2$.

In a particular embodiment, said naturally substantially non-porous material is selected from the group consisting of polyethylene terephthalate (PET), Polyethylene naphthalate (PEN), Poly(methyl methacrylate) (PMMA), Polyurethane (PU), Poly(vinyl chloride) (PVC), Polyethylene (PE), Polystyrene (PS), Polylactate, polyamide, and combinations thereof.

In another particular embodiment, the process of the invention further comprises a preliminary step, before step i), of preparing a substantially non-porous carrier starting from a porous material, comprising the steps of:

a) impregnating said porous material with at least one filler until saturation thereof; and
b) drying the impregnated material resulting from a).

In a particular embodiment, said porous material is cellulose or derivatives of cellulose such as for example nitrocellulose, aerogel, porous polymers or porous membranes such as for example PVDF membranes, PE membranes, Teflon membranes and Polycarbonate membranes, or non water soluble polysaccharides such as for example lignin, pectin, starch, dextran or chitosan.

In a particular embodiment, said at least one filler is selected in the group consisting of glucose, paraffin, sulfonated polymers, polyacrylic acid (PAA), poly-2-hydroxyethyl methacrylate (PHEMA), polymethyl methacrylate (PMMA), poly(poly(ethylene glycol) dimethacrylate) (PPEGDMA), polypropylene (PP), poly(styrene sulfonic acid - Maleic anhydride), poly(vinyl phosphonic acid), polyethyleneglycol, as well as salts thereof and/or combinations thereof.

In a particular embodiment, said biomolecule is selected from the group consisting of proteins or peptides, such as antibodies, antigens, enzymes, transcription factors, protein domains or binding proteins.

The present invention further concerns a grafted carrier obtained by the process of the invention, wherein said carrier comprises biomolecules immobilized thereonto.

The present invention further concerns a bioassay device comprising at least one carrier as defined above, wherein said bioassay device is preferably an immunoassay device such as an immunochromatographic strip or an immunochromatographic multiplex system.

The present invention further concerns the use of at least one carrier as disclosed above or of a bioassay device of the invention for diagnosis, affinity chromatography, proteomics, genomics and/or drug screening.

The present invention further concerns a bioassay kit comprising at least one carrier as defined above or at least one bioassay device of the invention.

## DETAILED DESCRIPTION

This invention therefore relates to a surprising and advantageous process allowing biomolecules to be immobilized on a substantially non-porous carrier and comprising the steps of (i) impregnating the carrier with a solution containing the said biomolecules; and (ii) irradiating the impregnated carrier resulting from step (i) with a light of a wavelength of at least 340 nm.

Within the present invention, by "impregnating", it is meant that biomolecules, in suspension in a solvent, are dispensed onto the substantially non-porous carrier by any method known in the art, such that the said biomolecules in solution are placed into contact with the surface of the carrier. The biomolecule solution may thus be dispensed onto the carrier under the form of drops, or droplets, such as for instance those formed by a printing system or by printing techniques such as silkscreen printing, inkjet printing, spraying, spin coating, and the like. In a preferred embodiment, the biomolecule solution is dispensed onto the carrier by inkjet printing. In another embodiment, the carrier is immerged in a solution comprising the said biomolecules.

Within the present invention, by "substantially non-porous carrier", it is meant that the carrier is substantially not permeable to water or to other fluids.

In a particular embodiment, the carrier use in the method of the invention is a naturally substantially non-porous carrier. In a particular embodiment, the naturally substantially non-porous carrier for use in the invention is selected in the group consisting of polyethylene terephthalate (PET), polyethylene naphthalate (PEN), poly(methyl methacrylate) (PMMA), polyurethane (PU), poly(vinyl chloride) (PVC), polyethylene (PE), polystyrene (PS), Polylactate, Polyamide (PA) and combination thereof.

In another embodiment of the invention, the carrier for use in the method of the invention is a naturally porous

carrier that has been pretreated for rendering it substantially non-porous. In a particular embodiment, suitable porous carriers for use in the present invention comprise cellulose or derivatives of cellulose such as for example nitrocellulose, aerogel, porous polymers or porous membranes such as for example PVDF membranes, PE membranes, Teflon membranes and polycarbonate membranes, or non water soluble polysaccharides such as for example lignin, pectin, starch, dextran or chitosan.

Within the present invention, by "cellulose", it is meant a polysaccharide of formula (a) (see below), consisting of a linear chain of several hundred to over ten thousand β(1-4) linked D-glucose units.

(a) 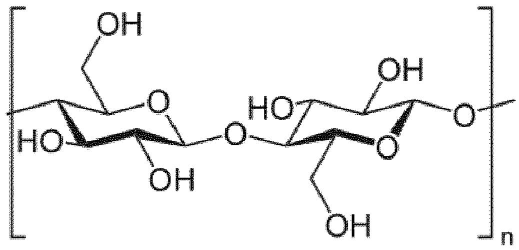

Cellulose for use within the present invention may be obtained from any origin, such as green plants, algae, oomycetes or *bacteria*. Cellulose for use in the present invention is non-functionalized, *i.e.* it has not been treated chemically for generating reactive moieties, nor coated with chemically- and/or photo-reactive intermediates. In a preferred embodiment, cellulose for use in the present invention is pure cellulose paper that has been pretreated for rendering it substantially non-porous. Cellulose provided under the form of a sheet is also known in the art under the term "paper".

In a particular embodiment, the said naturally porous carriers are rendered substantially non-porous by impregnating said carrier with at least one filling substance.

The present invention thus also concerns a process for preparing a substantially non-porous carrier starting from a porous material, and comprising the steps of:

a) impregnating said porous material with at least one filler until saturation thereof; and
b) drying the impregnated material resulting from a).

In a particular embodiment of the invention, step a) can be performed by any technique known in the art.

In a particular embodiment, the porous carrier is immerged in at least one filling substance solubilized in a suitable solvent. In a particular embodiment, the carrier is immerged for a period ranging from 1 hour to 24 hours. In another embodiment, the filling substance solubilized in a suitable solvent is allowed to flow through the carrier, e.g. under vacuum aspiration, until not outflow (or an extremely low outflow) is observed. In a particular embodiment, the carrier is impregnated with a solution comprising at least one filling substance or with a combination of filling substances. In a particular embodiment, the carrier is successively impregnated with several filling solutions comprising at least one filling substance or a combination of filling substances. In a particular embodiment, the carrier may be successively impregnated with filling substances solubilized in aqueous solvents and/or in organic solvents, provided that the organic solvents used for solubilizing the filling substance are water-miscible. In a particular embodiment, when filling substances are solubilized in water-immiscible organic solvents, the carrier is prealably impregnated with filling substances solubilized in aqueous solvents, then fully dried, before being impregnated with filling substances solubilized in water-immiscible organic solvents.

Within the present invention, by "filling substance" or "filler", it is meant a substance capable of substantially filling the pores of a porous carrier as defined above. Suitable fillers for use in the present invention comprise those that can be solubilized in an aqueous solvent, those that can be solubilized in a water-miscible organic solvent and those that can be solubilized in water-immiscible organic solvents. In a particular embodiment, fillers for use in the method of the invention are selected in the group consisting of glucose; paraffin; sulfonated polymers such as for example polystrene sulfonic acid and its derivatives, sulfonated polyacrylic acid and its derivatives; polyacrylic acid (PAA); poly-2-hydroxyethyl methacrylate (PHEMA); polymethym methacrylate (PMMA); poly(poly(ethylene glycol) dimethacrylate) (PPEGDMA); polypropylene (PP); poly(styrene sulfonic acid - Maleic anhydride); poly(vinyl phosphonic acid); polyethyleneglycol; as well as salts thereof and/or combinations thereof. In a preferred embodiment, glucose is used as a filler.

In a particular embodiment, when water-soluble substances are used for filling porous carriers such as cellulose, the concentration of such fillers ranges from 1 mg/mL to 1000 mg/mL, preferably about 100 mg/mL. In a particular embodiment, porous carriers saturated with water-soluble substances are impregnated for a time comprised between 0 min and 1 night. Water-soluble substance for use in the present invention is advantageously glucose.

In another particular embodiment, when water-miscible substances are used for filling porous carriers such as

cellulose, the concentration of such fillers ranges between 0.1 mg/mL and 1000 mg/mL. In a particular embodiment, porous carriers saturated with water-miscible substances are impregnated for a time comprised between 0 min and 1 night. Water-miscible substance for use in the present invention is advantageously polyacrylic acid, poly(styrenesulfonic acid-Maleic anhydride), or poly(ethyleneglycol).

In another particular embodiment, when water-immiscible substances are used for filling porous carriers such as cellulose, the concentration of such fillers ranges between 0.1 mg/mL and 100 mg/mL, preferably about 10 m/mL. In a particular embodiment, porous carriers saturated with water-miscible substances are impregnated for a time comprised between 0 min and 1 night. Water-immiscible substance for use in the present invention is advantageously paraffin or polystyrene.

Depending on the destination of the grafted carrier prepared according to the process of the present invention, the density and/or shape of the carrier may vary. Shapes suitable for the grafted carrier of the invention include but are not limited to: a bead, a well, a sheet, a powder, a stick, a plate, a strip, a tube, as well as any 3D shape alternative thereof.

Within the present invention, by "irradiating", it is meant subjecting the substantially non-porous carrier impregnated with the solution of biomolecules to a light having a wavelength of at least 340 nm. In a preferred embodiment, the light used for irradiating the impregnated carrier has a wavelength of from 340 nm to 800 nm (long-scale UV). In another embodiment, the light used for irradiating the impregnated carrier has a wavelength of from 400 nm to 800 nm. In a preferred embodiment, the light used has a wavelength of about 365 nm.

Within the process of the invention, irradiation is conducted for a period of time and with a wavelength that are suitable for subjecting the impregnated carrier to a photoenergy of from 1 mJ/$cm^2$ to 500 J/$cm^2$, preferably of from 1 J/$cm^2$ to 80 J/$cm^2$. In a preferred embodiment, irradiation is conducted for a period of time and with a wavelength that are suitable for subjecting the impregnated carrier to a photoenergy of about 10 J/$cm^2$.

Within the process of the invention, irradiation is conducted for a period of time of at least 1 second, preferably from 16 to 1280 minutes, preferably of about 160 minutes.

Within the present invention, by "immobilized", it is meant that moieties of the biomolecules of interest present in the solution used for impregnating the substantially non-porous carrier are covalently coupled to moieties of the carrier surface, by co-chemical reaction triggered by irradiation. As a result of the process of the invention, the biomolecules of interest are thus covalently coupled to the carrier, while having preserved 80%, preferably 90%, preferably 99% and more preferably 100% of their biological activity.

Within the present invention, by "biomolecule", it is meant any biological molecule selected from the group consisting of proteins or peptides, such as antibodies, antigens, enzymes, transcription factors, protein domains or binding proteins. In a preferred embodiment, the biomolecules for use in the present invention are antibodies and proteins. In a preferred embodiment, the biomolecules for use in the present invention are native biomolecules, *i.e.* they are not modified either through chemical processes or by genetic engineering for improving their cellulose binding properties. In another preferred embodiment, the biomolecules for use in the present invention are modified, for example through the addition of a labelling system. In a preferred embodiment, the biomolecules of interest may correspond to biomolecules involved into biological membranes such as transmembrane proteins or antigenic proteins or peptides displayed on a biological membrane. In a particular embodiment of the present invention, the biomolecules of interest may be involved in the biological membrane of a cell, in the outer membrane or in the cell wall of a *bacteria* or a virus. In a preferred embodiment, by a solution of "biomolecules", it is also meant a solution wherein the biomolecules of interest are provided on the membrane, cell wall or envelope of a cell, a *bacteria* or a virus, together with the said cell, *bacteria* or virus. In another embodiment, the biomolecules for use in the present invention correspond to biomolecules which are not excreted by cells such as non-excreted proteins. In another embodiment, the biomolecules for use in the present invention are free in solution, and may have been either purified from a biological organism or synthesized *in vitro.* For use in the method of the invention, the biomolecules are solubilized in a solvent including but not limited to: water, water for injection, phosphate buffer, carbonate buffer, borate buffer, HEPES buffer, MES buffer or any other aqueous biological buffer.

Within the present invention, by "drying", it is meant evaporating the solvent wherein the biomolecules of interest are solubilized, thus allowing the corresponding biomolecules to be concentrated on the surface of the carrier. In a preferred embodiment, the carrier impregnated with the solution containing the biomolecules of interest is dried before the irradiation step. Drying may be performed by any mean known in the art, and in particular in subjecting the impregnated carrier to a source of heating, such as for instance, a ventilated oven.

In a particular embodiment of the present invention, a drying step is implemented before the irradiation step when said irradiation step (iii) is performed with a photoenergy of at least 1 mJ/$cm^2$, preferably of at least 1 J/$cm^2$, and preferably of about 10 J/$cm^2$.

Within the present invention, by "washing", it is meant applying a liquid on the irradiated carrier (*i.e.* after the irradiation step) for removing any unbound biomolecule. According to the process of the invention, the washing step may be performed with any solvent which does not alter the structure or the biological activity of the immobilized biomolecules and which does not alter the carrier. Preferred washing solutions for the use in the present invention include but are not limited to: water, water for injection, phosphate buffer, carbonate buffer, borate buffer, HEPES buffer, MES

buffer or any other aqueous biological buffer, optionally enriched with salts and/or detergent.

Another object of the present invention concerns a grafted carrier obtained by the process of the invention wherein said carrier comprises biomolecules immobilized thereonto.

Within the present invention, by "grafted carrier", it is meant a substantially non-porous carrier wherein biomolecules of interest were immobilized through the process of the invention, *i.e*. though irradiation at a wavelength of at least 340 nm. As a result of the process of the invention, the said grafted carrier is thus composed of biomolecules of interest which are covalently linked to a carrier absent any chemical intermediate or adjuvant, and absent any functionalization of the said biomolecules.

Another object of the present invention concerns a bioassay device, preferably an immunoassay device, such as for instance an immunochromatographic strip or an immunochromatographic multiplex system.

Within the present invention, by "bioassay device", it is meant any device intended for detecting and/or quantifying biological or non-biological compounds, objects or organisms, including at least one grafted carrier of the invention.

Within the present invention, by "immunoassay device", it is meant any device intended for immunodetection or immunoquantification purposes including at least one grafted carrier of the invention.

Within the present invention, by "immunochromatographic strip", it is meant a device composed of a loading area, a detection area and an absorbent pad, the whole being affixed onto a plastic carrier. The detection area is formed by grafted carriers according to the invention. Migration is supported by two migration areas, surrounding the detection area, that are capable to trigger the sample to be tested by adsorption to the detection area.

Within the present invention, by "immunochromatographic multiplex system", it is meant a system generally composed of hydrophilic paper channels, delimited by hydrophobic plastic stencils, such as those disclosed in patent applications filed under Nos. FR1161722 and FR1260083. The several levels of the system are stacked and connected together for forming a 3D fluidic system. A sample introduced at the top of the system is divided in as many aliquots as the number of channels, and is thus allowed to contact various biomolecules of interest immobilized on cellulose carriers according to the invention, and displayed in separated assay areas. An immunochromatographic multiplex system for instance allows different antigens to be detected within a same sample, using several carriers grafted with different antibodies.

Another object of the present invention concerns the use of at least one carrier according to the invention or of an immunoassay device according to the invention for diagnosis, affinity chromatography, proteomics, genomics and/or drug screening.

## BRIEF DESCRIPTION OF THE DRAWINGS

**Figure 1** is a drawing representing schematically a printed pattern as implemented for the experiences disclosed herein.

**Figure 2** is a drawing representing schematically the structure (a) and the proportions (b) of an immunochromatographic strip.

**Figure 3** is a photograph showing the results of a Photo-patterning assay conducted with gold-labeled goat anti-mouse tracer antibodies. Photographs were taken with a regular digital camera.

**Figure 4** corresponds to Photographs showing the influence of the dispensing process on biological activity and membrane VDL. The first set of strips (a) results from usual BioDot dispensing method, the second (b) from 1-layer inkjet printing, and the third (c) from 5-layer inkjet printing. Antibodies were adsorbed onto nitrocellulose and photoimmobilized onto cellulose. Their actual immobilization was confirmed thanks to gold-labeled goat anti-mouse tracer (control strips). The capture of OVA antigen by the immobilized antibodies was highlighted by gold-labeled murine anti-OVA tracer (OVA strips). The strips corresponding to the membranes VDL are labeled with a cross.

Photographs were taken with the Molecular Imager. All experiments were reproduced 3 times but only one is shown here.

**Figure 5** is a drawing representing the detailed structure of an IgG antibody molecule (a) and the general structure of an IgM antibody molecule (b).

**Figure 6** is a graph showing the variations of antibody solutions viscosities at 24°C as a function of shear rate varying from 100 to 10 000 $s^{-1}$. The plain line corresponds to murine monoclonal antibody anti-OVA. The discontinued

line corresponds to goat polyclonal antibody anti-mouse.

**Figure 7** is a drawing representing the molecular structure of the paper substrates (a) and filling substances (b) used in the examples.

**Figure 8** is a graph representing the XPS survey analysis of unprinted paper substrates. (a) is spectrum from nitrocellulose sheet, (b) from cellulose, (c) from glucose-cellulose and (d) from paraffin-cellulose. The peaks corresponding to O 1s, C 1s and N 1s orbitals are labeled.

**Figure 9** is a graph showing the IR spectra of unprinted paper substrates. (a) is spectrum from nitrocellulose sheet, (b) from cellulose, (c) from glucose-cellulose and (d) from paraffin-cellulose. All spectra have several bands in common which correspond to O-H, C-H, C-C, C-O and O-C-O stretching vibrations. The N-O stretching vibrations specific to nitrocellulose are labeled.

**Figure 10** is a graph showing the line profiles of the unprinted paper substrates.

**Figure 11** is a histogram showing the comparative Surface roughness (Ra) of the unprinted paper substrates.

**Figure 12** is a photograph showing SEM micrographs of unprinted nitrocellulose (a), cellulose (b), glucose-cellulose (c) and paraffin cellulose (d).

**Figure 13** is a graph showing the XPS survey analysis of antibody-printed paper substrates. (a) is spectrum from nitrocellulose sheet, (b) from cellulose. The peaks corresponding to O 1s, C 1s and N 1s orbitals are labeled.

**Figure 14** is a graph showing the IR spectra of antibody-printed paper substrates. (a) is spectrum from nitrocellulose sheet, (b) from cellulose, (c) from glucose-cellulose and (d) from paraffin-cellulose. All spectra have several bands in common which correspond to O-H, C-H, C-C, C-O and O-C-O stretching vibrations. The N-O stretching vibrations specific to nitrocellulose are labeled.

**Figure 15** is a Photograph showing the influence of the substrate and its pretreatment on biological activity and membrane VDL. The first set of strips (a) is made of nitrocellulose, the second (b) of cellulose, the third (c) of glucose-cellulose and the fourth (d) of paraffin-cellulose. Antibodies were adsorbed onto nitrocellulose and photoimmobilized onto cellulose-based substrates. Their actual immobilization was confirmed thanks to gold-labeled goat anti-mouse tracer (control strips). The capture of OVA antigen by the immobilized antibodies was highlighted by gold-labeled murine anti-OVA tracer (OVA strips). The strips corresponding to the membranes' VDL are labeled with a cross. Photographs were taken with the Molecular Imager. All experiments were reproduced 3 times but only one is shown here.

**Figure 16** is a photograph showing the biological activity of antibodies printed according to a complex design. The first set of strips (a) was produced with nitrocellulose membrane, the second (b) with cellulose. Antibodies were adsorbed onto nitrocellulose and photoimmobilized onto cellulose. The capture of OVA antigen by the immobilized antibodies was highlighted by gold-labeled murine anti-OVA tracer. For each set of strips photographs were taken with both a digital camera (colored left pictures) and the Molecular Imager (grey right pictures).

Figure 17 is a SEM photograph representing untreated CF1 cellulose sheet (see A)) and CF1 sheet treated with a solution of Poly(acrylic acid) sodium salt (250.000 (Sigma Aldrich)).

**Figure 18** is a SEM photograph representing a x50 magnification (A) or a x300 magnification (B) of a PET surface wherein a) corresponds to the virgin PET surface, b) corresponds to proteins printed on PET, c) corresponds to protein-printed PET surface washed with ultrasound for 10s, and d) corresponds to protein-printed PET surface washed with ultrasound for 10 min.

**Figure 19** is a graph showing the IR result obtained with virgin PET, protein-printed PET, protein-printed PET washed with water and with ultrasound for 10s or 10 min.

EXAMPLES

The present invention is further illustrated by the following examples.

### *Materials and Methods*

Materials

Proteins (ovalbumin (OVA), Bovine Serum Albumin (BSA) and porcine skin gelatin), as well as chemical products for preparing buffers, colloidal gold solution, and substrates pretreatment mixtures were obtained from Sigma-Aldrich (St Louis, MO, USA). Water used in all experiments was purified by the Milli-Q system (Millipore, Brussels, Belgium). Monoclonal murine antibodies (murine mAbs) were produced at LERI (CEA, Saclay, France) as described by Khreich et al. (Khreich et al., Toxicon 2009, 53, 551-559). Goat anti-mouse antibodies (IgG + IgM (H+L)) were purchased from Jackson ImmunoResearch (West Grove, PA, USA).

Papers used for preparing the immunoassay membranes were CF1 cellulose and AE 98 Fast nitrocellulose from Whatman (Maidstone, Kent, UK), and printing paper Xerox premier 80 (Ref. 3R91720, Xerox, Norwalk, CT, USA). Immunochromatographic strips were prepared using Standard 14 sample wick from Whatman (Maidstone, Kent, UK), No. 470 absorbent pad from Schleicher and Schuell BioScience GmbH (Dassel, Germany) and MIBA-020 backing card from Diagnostic Consulting Network (Carlsbad, CA, USA).

Antibody solutions were either printed onto substrates using a laboratory piezoelectric drop-on-demand inkjet printer Dimatix Materials Printer DMP-2831 (Fujifilm, Santa Clara, CA, USA) with 10 pL nominal drop volume cartridge, or dispensed at 1 $\mu L\ cm^{-1}$ using an automatic dispenser (XYZ3050 configured with 2 BioJet Quanti Dispenser (BioDot, Irvine, CA, USA)). Irradiations were conducted at room temperature in a UV chamber CN-15.LV UV viewing cabinet (Vilber Lourmat, Marne-la-Vallee, France). Strips were cut using an automatic programmable cutter Guillotine Cutting CM4000 Batch cutting system from BioDot (Irvine, CA, USA). 96-Well polystyrene microplates (flat-bottom, crystal-clear, from Greiner Bio-One S.A.S. Division Bioscience, Les Ulis, France) were used as container for migrations on immunochromatographic strips. Opaque plastic (double-sided tape) maskings used in the photo-patterning experiments have been designed and prepared with a laser plotter LaserPro Spirit (GCC Laser Pro, New Taipei City, Taiwan), and the software CorelDRAW Graphics Suite (Corel Corporation, Ottawa, Canada).

Characterization materials

Infrared (IR) spectra of the various substrates were recorded on a Vertex 70 FT-IR spectrometer (Bruker, Billerica, MA, USA) controlled by OPUS software (Bruker, Billerica, MA, USA) and fitted with MIRacle™ ATR (Attenuated Total Reflectance) sampling accessory (PIKE Technologies, Madison, WI, USA). The ATR crystal type was single reflection diamond/ZnSe crystal plate. The FT-IR detector was MCT working at liquid nitrogen temperature. Acquisitions were obtained at 2 $cm^{-1}$ resolution after 256 scans.

X-ray photoelectron spectroscopy (XPS) studies of membranes were performed with an Axis Ultra DLD spectrometer (Kratos, Manchester, UK), using monochromatic Al $K_\alpha$ radiation (1486.6 eV) at 150 W and a 90° electron take-off angle. The area illuminated by the irradiation was about 2 mm in diameter. Survey scans were recorded with 1 eV step and 160 eV analyzer pass energy and the high-resolution regions with 0.05 eV step and 40 eV analyzer pass energy. During the data acquisition, the sample surfaces were neutralized with slow thermal electrons emitted from a hot W filament and trapped above the sample by the magnetic field of the lens system (hybrid configuration). Referring to Johansson and Campbell's work, XPS analysis was carried out on dry samples, together with an *in situ* reference (Johansson and Campbell, Surf. Interface Anal. 2004, 36, 1018-1022).

Microstructure and surface morphology of samples were examined by a JSM-5510LV (JEOL, Tokyo, Japan) scanning electron microscope (SEM) after gold coating (K575X Turbo Sputter Coater (Quorum Technologies Ltd, Ashford, Kent, UK), working at 15 mA for 20 seconds). The images were acquired at various magnifications ranging from 100x to 3 000 ×. The acceleration voltage and working distance were 4 kV and 17 mm, respectively. Images were acquired applying the secondary electron detector.

Surface roughness, Ra, of the unprinted substrates was measured with an AlphaStep® D-120 Stylus Profiler (KLA-Tencor, Milpitas, CA, USA). Measurements were performed along a line of 1 mm long, with a stylus force of 1 mg and at a speed of 0.05 mm $s^{-1}$.

Printed solutions viscosity was measured before printing with a MCR 102 Rheometer (Anton Paar, Ashland, VA, USA). Cone-plane geometry was used at a shear rate varying from 100 to 10 000 $s^{-1}$ and at a 24°C temperature. Gap distance was equal to 0.1 mm. Geometry diameter and angle were equal to 5 cm and 1°, respectively.

Colorimetric intensity resulting from colloidal gold on immunochromatographic strips was qualitatively estimated directly by eye at first and then indirectly through a picture taken with a Molecular Imager VersaDoc™ MP4000, in association with Quantity One 1-D Analysis software (Bio-Rad, Hercules, CA, USA). Colorimetric intensity resulting from colloidal gold on masked papers was quantified with the same imager and software.

Substrates pretreatments

AE 98 Fast nitrocellulose, CF1 cellulose and Xerox premier cellulose were used as received. Several pieces of CF1 cellulose and Xerox premier cellulose were treated in order to obtain cellulose sheets enriched with glucose (glucose-cellulose), paraffin (paraffin-cellulose) or with various chemical fillers (see in particular examples 8 to 14).
Glucose-cellulose was prepared by dipping a CF1 cellulose sheet in a 100 mg $mL^{-1}$ aqueous solution of D-(+)-glucose overnight at 4°C, and then drying it at 37°C in a ventilated oven for 1 hour.
Similarly, paraffin-cellulose was prepared by dipping a cellulose sheet in a 10 mg $mL^{-1}$ hot aqueous suspension of paraffin for 1 hour, and then drying it at 37°C in a ventilated oven for 1 hour. The temperature of the aqueous solution needed to be above 60°C for paraffin to melt and mix with water.
Method implemented in examples 8 to 14 for pretreating cellulose sheets and rendering

them substantially non-porous

A sheet of cellulose was placed on a sintered glass or on a Millipore filtration system (suitable for filtering on polymer membranes) possessing a metallic grid basis with a great porosity. This assembly was mounted on a vacuum flask and vacuum was applied. Various filling solutions, prepared with the following polymers:

- Poly(acrylic acid) sodium salt, 35% in $H_20$ Mw 60.000 (PolySciences Inc);
- Poly(acrylic acid) sodium salt, 35% in $H_20$ Mw 250.000 (Sigma Aldrich);
- Poly(acrylic acid) powder Mw 1.000.000 (PolySciences Inc);
- Poly(styrenesulfonic acid-Maleic anhydride), 3 :1, Low Mn (Polysciences Inc);
- Poly(vinylphosphonic acid), 30% in $H_20$;
- PolyStyrene solid, Mw 192.000 (Sigma Aldrich); and
- Poly(ethyleneglycol) Mw 950-1.050 (Sigma Aldrich),

were loaded onto a cellulose sheet, then allowed to flow through the sheet under vacuum suction. The polymer solutions were thus penetrated into the cellulose sheet until saturation, i.e. until no outflow (or an extremely low outflow) was observed.

The thus-obtained cellulose sheets were then dried at room temperature for 24 to 48 hours.
Solvents for preparing the polymer solutions were selected in accordance with the chemical nature of the tested polymers, such as to ensure the solubility thereof. Suitable solvents were aqueous, e.g. water was used for solubilizing Poly(acrylic acid) sodium salts, or organic, e.g. acetone was used as solvent for solubilizing polystyrene. In a first embodiment, cellulose sheets were impregnated with a solution containing a single polymer, or containing a mixture of at least two polymers. In another embodiment, cellulose sheet were impregnated by successive filtering with various polymer solutions. Impregnations conducted with aqueous polymer solutions could be interspersed with impregnations conducted with organic polymer solutions provided that:

i) organic solvents used for solubilizing polymers were water-miscible (such as, for instance, Tetrahydrofuran (THF), Dimethylformamide (DMF), acetone, ethanol, etc.); or
ii) cellulose sheets filtered with aqueous polymer solutions were fully dried before impregnation with water-immiscible organic polymer solutions (e.g. polystyrene solutions).

Immobilization procedure

*Printing*

Antibody solutions were printed onto the raw and pretreated substrates using the Dimatix inkjet printer. Nozzle diameter was 21.5 μm and nominal drop volume was 10 pL. Printing tests were performed at 40 V tension with 15 μm drop spacing. While drop spacing is inversely proportionate to resolution, printing voltage is directly related to the ejected volume. The printed pattern (see Figure 1) consisted of two straight lines of 600 μm width and was designed according to usual LFIA strips (Khreich et al., Anal. Biochem. 2008, 377, 182-188). The bottom line was therefore dedicated to capture the OVA model antigen (test line). The top line aimed to detect anti-OVA tracer antibodies (control line). Thus, the test line consisted of murine anti-OVA monoclonal antibodies (1 mg $mL^{-1}$ in 0.1 M potassium phosphate buffer, pH 7.4) and the control line of goat anti-mouse polyclonal antibodies (0.5 mg $mL^{-1}$ in 0.1 M potassium phosphate buffer, pH 7.4). Printings made of 1 and 5 layers were compared to the usual automatic dispensing method (1μL $cm^{-1}$ with the BioDot system) (Khreich et al., Anal. Biochem. 2008, 377, 182-188).

*Immobilization*

Two procedures were implemented depending on the nature of the substrate. Thus, antibodies were adsorbed onto nitrocellulose substrate (AE 98 Fast nitrocellulose), while they were photoimmobilized onto cellulose substrates (CF1 or Xerox premier cellulose, glucose-cellulose and paraffin-cellulose) or onto PET substrates. Results obtained onto the raw and pretreated cellulose substrates or PET substrates were analyzed with respect to nitrocellulose as the reference material.

The photoimmobilization process for antibody immobilization onto cellulose, pretreated cellulose or PET substrate can be described as follows: (i) an antibody solution was dispensed onto a substrate sheet formed of cellulose, of pretreated cellulose or of any naturally substantially non-porous material (e.g. PET); (ii) antibodies were concentrated by drying of the impregnated substrate at 37°C, in a ventilated oven, for 15 minutes; (iii) the system was irradiated at 365 nm (1050 $\mu W\ cm^{-2}$) for 2h40 (about 10 $J\ cm^{-2}$) for inducing photoimmobilization; and (iv) substrates were intensively rinsed with a washing buffer (0.1M potassium phosphate buffer, pH 7.4, containing 0.5 M NaCl and 0.5% (v/v) Tween 20) for removing non-immobilized antibodies.

Adsorption of antibodies onto nitrocellulose was achieved by regular 1-hour incubation at room temperature and following washing step.

<u>Immunochromatographic assays (LFIA)</u>

Immobilization rate, biological activity and visual detection limit (VDL) of the antibody-printed membranes were evaluated by colloidal-gold-based lateral flow immunoassays (LFIAs) (Ngom et al., J. Mater. Chem. B 2013, 1, 3277-3286). The signal intensity was qualitatively estimated directly by eye at first and then indirectly through a picture taken with a Molecular Imager. All results were compared with those obtained with nitrocellulose which is the reference material.

All the reagents were diluted in the analysis buffer (0.1 M potassium phosphate buffer, pH 7.4, containing 0.1% (w/v) BSA, 0.15 M NaCl, and 0.5% (v/v) Tween 20), at room temperature, 30 minutes prior to migration in order to reduce nonspecific binding. Each assay was performed at room temperature by inserting a strip into a well of a 96-well microtiter plate containing 100 μL of the test solution. The mixture was successively absorbed by the various pads and the capillary migration process lasted for about 15 minutes. Colorimetric intensity was immediately estimated by eye and pictures with both regular digital camera and Molecular Imager were taken without delay.

*Preparation of colloidal-gold labeled antibodies*

Tracer antibodies were labeled with colloidal gold according to a known method previously described (Khreich et al. Anal. Biochem. 2008, 377, 182-188). Two types of tracer were prepared: a goat anti-mouse tracer to reveal the immobilized murine antibodies, and a murine anti-OVA tracer to highlight the capture of OVA by the immobilized antibodies.

Briefly, 4 mL of gold chloride and 1 mL of 1% (w/v) sodium citrate solution were added to 40 mL of boiling water under constant stirring. Once the mixture had turned purple, this colloidal gold solution was allowed to cool down to room temperature and stored at 4°C in the dark. 25 μg of mAb and 100 μL of 20 mM borax buffer, pH 9.3, were added to 1 mL of this colloidal gold solution. This mixture was left to incubate for one hour on a rotary shaker at room temperature, therefore enabling the ionic adsorption of the antibodies onto the surface of the colloidal gold particles. Afterwards, 100 μL of 20 mM borax buffer, pH 9.3, containing 1% (w/v) BSA, was added and the mixture was centrifuged at 15 000 g for 50 minutes at 4°C. After discarding the supernatant, the pellet was suspended in 250 μL of 2 mM borax buffer, pH 9.3, containing 1% (w/v) BSA and stored at 4°C in the dark.

*Preparation of immunochromatographic strips*

An immunochromatographic strip is usually composed of a sample pad, a detection pad and an absorbent pad, the whole being affixed onto a plastic carrier (or backing card). Thus, an antibody-printed paper pad constituted the detection zone. In order to prevent nonspecific protein adsorption onto the detection membrane during immunoassays, all antibody-printed membranes were saturated with a gelatin solution (0.1 M potassium phosphate buffer, pH 7.4, containing 0.5% (w/v) porcine gelatin and 0.15 M NaCl) overnight at 4°C, and then dried at 37°C in a ventilated oven for 30 minutes. All pads (about 20 cm width) were assembled onto the backing card and then the whole was cut into strips of 5 mm width (see Figure 2).

*Assessment of the immobilization*

The test solution was composed of a goat anti-mouse tracer diluted 10 times in the analysis buffer. Unprinted parts of detection paper pads assessed the unspecific signal due to unspecific adsorption of the tracer onto the saturating matrix during immunoassays. The immobilization ability of the various paper substrates was therefore assessed by the colorimetric difference between the murine-antibody-printed part of detection pad (test line) and the unprinted corresponding one.

*Assessment of the biological activity and determination of the visual detection limit*

Ten test solutions were prepared and pre-incubated for 15 minutes. The first one only contained murine anti-OVA mAb tracer diluted 10 times in the analysis buffer. This immunoassay without OVA antigen (0 ng $mL^{-1}$) assessed the unspecific signal due to unspecific adsorption of the tracer onto the antibody-gelatin matrix during immunoassays (negative control). The nine others were solutions of murine anti-OVA mAb tracer (10-time dilution) and OVA (dilution series ranging from 1 ng $mL^{-1}$ to 500 ng $mL^{-1}$) in the analysis buffer.

The biological activity of the various paper substrates was therefore assessed by the colorimetric difference between the antibody-printed paper test-line signal in the presence of OVA and the corresponding one without OVA. Since it captured the excess murine anti-OVA tracer antibodies, the control line prevented false negative results. Its coloring guaranteed that the tracer actually passed through the test line, along with the test solution.

The visual detection limit (VDL) was determined through the OVA dilutions series. It was defined as the minimum OVA concentration resulting in a test-line colored signal significantly more intense than the negative control one.

<u>Patterned photoimmobilization of probe antibodies</u>

Probe antibodies, or colloidal-gold-labeled antibodies (tracers), were photoimmobilized onto pristine CF1 cellulose paper according to the following procedure. A 2-$cm^2$ cellulose sheet (2 cm x 1 cm in size) was manually impregnated with a goat anti-mouse tracer solution (3-fold dilution in the analysis buffer, 20 $\mu$L $cm^{-2}$ deposit). Drying step was skipped and this system was then irradiated at 365 nm for 1h20 (about 5 J $cm^{-2}$) through an opaque plastic mask in order to localize the grafting (patterning process). Paper was rinsed overnight with the washing buffer. Colorimetric measurement using the molecular imager was performed immediately after the paper had been slightly dried over absorbent paper. The patterned image was pictured with either digital camera or VersaDoc™ Molecular Imager.

<u>Inks: composition</u>

Printed solutions, also called inks, were antibody aqueous solutions. Because of different initial proportions in each antibody stock solution, their final salts content was different. Thus, murine anti-OVA antibody solution (test line ink) actually contained 1 mg $mL^{-1}$ of monoclonal antibody (IgG) and 0.1 M of potassium phosphate in water. Likewise, goat anti-mouse antibody solution (control line ink) contained 0.5 mg $mL^{-1}$ of polyclonal antibody (IgG + IgM), 0.1 M of potassium phosphate and 0.05 M of sodium chloride (NaCl). These variations in salts content, but also in antibody type (IgG and IgM structures are depicted in Figure 5) could greatly influence the surface tension between the antibody ink and the paper substrate, thereby inducing variations in the printing behavior.

*Initial substrates: molecular structure*

Cellulose is a natural biopolymer made up of glucose units (Figure 7a). It is the simplest polysaccharide since it is composed of a unique monomer (glucose) which binds to its neighbors by a unique type of linkage ($\beta$-1,4 glycosidic bond resulting in acetal function). According to its molecular structure, hydroxyl groups in glucose units are responsible for cellulose chemical activity (Roy et al., Chem. Soc. Rev. 2009, 38, 2046-2064). However, this group cannot directly interact with proteins, what usually makes cellulose activation or functionalization necessary in order to covalently bind to proteins of interest.

Cellulose pretreatments as described herein introduced few additive molecules but did not change the native chemical structure of cellulose. Additive substances were adsorbed onto it and partially filled its pores. These additives were glucose, paraffin, as well as Poly(acrylic acid), Poly(styrenesulfonic acid-Maleic anhydride), Poly(vinylphosphonic acid), PolyStyrene and Poly(ethyleneglycol). While glucose is the molecular repeating unit in cellulose macromolecule, paraffin is a mixture of linear alkanes (see Figure 7b).

Nitrocellulose (also named cellulose nitrate) is the most important cellulose derivative. Biomolecules strongly adsorb to nitrocellulose through a combination of electrostatic, hydrogen, and hydrophobic interactions involving the nitro functions. It is therefore the reference material for performing lateral flow immunoassay (LFIA) (Posthuma-Trumpie

et al., Anal. Bioanal. Chem. 2009, 393, 569-582; Ngom et al., Anal. Bioanal. Chem. 2010, 397, 1113-1135; Fridley et al., MRS Bull. 2013, 38, 326-330).

Cellulose nitrate is formed by esterification of hydroxyl groups from cellulose (primary or secondary) with nitric acid in the presence of sulfuric acid, phosphoric acid or acetic acid (see Figure 7a) (Roy et al., Chem. Soc. Rev. 2009, 38, 2046-2064).

These molecular features represent the first, but not most, difference between the nitrocellulose and cellulose-based substrates.

Example 1: Localized immobilization of probe antibodies

Photo-patterning consists in transferring an image displayed on a mask towards a substrate through photochemical or photoactivated reactions. This is the fastest and most easily undertaken process ensuring the localization of species onto a flat support according to a well-defined and reproducible pattern. This process was therefore combined to the above disclosed chemical-free photografting procedure in order to easily and rapidly localize antibodies onto cellulose sheets.

Probe antibodies labeled with colloidal gold were immobilized through a mask in order to directly observe the photo-patterned immobilization of antibodies, and to evaluate the signal/background *ratio* (Figure 3). A selective photoimmobilization of the colloidal-gold-labeled antibody is observed according to the design of the used mask. This confirms the immobilization process to be photo-controlled. The signal/background *ratio* is estimated to be around 140%. Though it is a rather positive result, the high background colorimetric intensity also indicates that lots of antibodies are wasted in this process. That stems from the subtractive nature of the photo-patterning process. Thus, this process was set aside and an additive process such as inkjet printing was further preferred.

Example 2: From classical automatic dispensing to inkjet printing of antibodies

Since automatic dispensing with BioDot-like systems (Khreich et al., Anal. Biochem. 2008, 377, 182-188) is the most frequently used method for antibody dispensing onto immunoassay membranes, the inkjet printing approach was first compared to the latter. That comparison aimed to validate the printing method for its use in the development of immunoassay devices.

Printings made of 1 and 5 layers were therefore compared to the single line deposit from the automatic dispenser (Figure 4). After antibody solutions had been dispensed onto the substrates, the antibodies were either adsorbed onto nitrocellulose or photoimmobilized onto cellulose. First, their immobilization was confirmed by revelation with gold-labeled goat anti-mouse tracer (see control strips in Figure 4). Then, their biological activity was put to the test by exposition to OVA antigen and simultaneously revealed by gold-labeled murine anti-OVA tracer (sandwich immunoassay) (see OVA strips in Figure 4). Each test was performed in triplicate.

The first noticeable result is that the sets of strips obtained with BioDot dispensing method and with 5-layer inkjet printing are visually almost identical. Their coloring is quite strong, while the coloring resulting from 1-layer inkjet printing is obviously weaker. However, this weakness does not seem to lower its performances in terms of visual detection limit (VDL) as further detailed. This same set of strip actually displays slightly thinner and more precise test and control lines than the others, although they are all well-defined, thin and precise. With regard to biological activity, dilutive effect is clearly perceptible. Nevertheless, photographs reveal that the negative control (OVA at 0 ng $mL^{-1}$) for nitrocellulose is slightly colored. This raises the issue of false positive results that can be observed with nitrocellulose immunoassay membranes. This issue does not arise with cellulose, most probably because of lower sensitivity. Considering that, the membranes' VDL were appraised as follows: (i) 5 ng $mL^{-1}$ for nitrocellulose and 25 ng $mL^{-1}$ for cellulose with BioDot dispensing method (Figure 4a); (ii) 1 to 5 ng $mL^{-1}$ for nitrocellulose and 25 ng $mL^{-1}$ for cellulose with 1-layer inkjet printing (Figure 4b); and (iii) 1 to 5 ng $mL^{-1}$ for nitrocellulose and 25 ng $mL^{-1}$ for cellulose with 5-layer inkjet printing (Figure 4c).

Each material VDL was therefore identical regardless the dispensing method or the number of layers. Thus, the printing process was indeed proved to be as efficient as the usual automatic dispensing, and therefore totally legitimate regarding its use in the development of immunoassay devices. Moreover, the printing method has the advantage of saving the quite expensive biomolecules dispensed because of the rather low ejected volume. Though an exact ejected volume could not be measured, the maximum dispensed volume was calculated based on the printer features (nominal drop volume, drop spacing and tension). For the selected pattern (a straight line of 600 $\mu$m width), the printer was estimated to deliver 0.27 $\mu L\ cm^{-1}$ of antibody solution per layer. A maximum of 0.27 $\mu L\ cm^{-1}$ of antibody solution was thus dispensed with 1-layer inkjet printing (Figure 4b), a maximum of 1.35 $\mu L\ cm^{-1}$ with 5-layer inkjet printing (Figure 4c), and exactly 1 $\mu L\ cm^{-1}$ with BioDot dispensing method (Figure 4a). Since a 1-layer printing is efficient enough to determine the VDL, the consumed amount of antibodies is therefore nearly a quarter of the amount consumed with a classical automatic dispenser. Another advantage of printing over classical automatic dispensing is the freedom in design of the printed pattern (see below) while the usual automatic dispenser only allows drawing straight lines of rather undefined

width.

Regarding the evaluation of the immobilization procedure, photoimmobilization onto cellulose led to VDL results in the same order of magnitude as the values obtained with adsorption onto nitrocellulose. However, cellulose performances appeared slightly lower than nitrocellulose's ($VDL_{cellulose}$=5 $VDL_{nitrocellulose}$). Beyond procedure, this phenomenon might stem from the many differences both chemical and physical between the two substrates. This is why the experiments presented thereafter were dedicated to characterize these differences while trying to compensate for them by cellulose pretreatment.

Example 3: Inkjet printing of antibodies onto various substrates

Beyond the obvious chemical difference in molecular structure, the main physical difference between nitrocellulose and cellulose substrates lies in their porosity (about 5 μm and 11 μm surface pore size, respectively) and sheet thickness (20 μm and 176 μm thick, respectively). Since cellulose sheets with same porosity and thickness than nitrocellulose were not commercially available, cellulose pretreatments which aimed to compensate for that by filling cellulose pores were achieved. Given that the filling substance should be inert regarding antibody immobilization process and further immunoassays, two components were initially selected: glucose and paraffin. Glucose is the molecular repeating unit in cellulose macromolecule (see Figure 7a and b) and therefore was not expected to disturb the immobilization process or further use of the membrane. In addition, its high water solubility (180 mg $mL^{-1}$) would permit to easily remove it during post-irradiation washing step. Paraffin, a mixture of linear alkanes (see Figure 7b), is well known for its unreactive nature (Noh et al. Anal. Chem. 2010, 82, 4181-4187). Unlike glucose, it is insoluble in water and therefore would stick into the fibers after the washing step and during further immunoassays.

Antibody solutions were printed onto the raw (nitrocellulose and cellulose) and pretreated (glucose-cellulose and paraffin-cellulose) substrates. Though 1 layer would have been enough, 5 layers were actually printed in order to get strong color intensity. Antibodies were then adsorbed onto nitrocellulose substrate and photoimmobilized onto cellulose substrates (cellulose, glucose-cellulose and paraffin-cellulose). Surface morphological structure and chemical composition of both raw and pretreated substrates were analyzed prior to printing and afterwards. Printed antibody solutions were characterized as well. Finally, lateral flow immunoassays (LFIAs) ensured the ultimate characterization by evaluating the biological activity and visual detection limit of the various membranes.

The viscosity of both test line and control line antibody solutions was measured (Figure 6). As reminded above, test line ink consisted of murine anti-OVA monoclonal antibodies and control line ink of goat anti-mouse polyclonal antibodies. According to Figure 6, control line ink viscosity varies from 2.28 to 1.69 mPa s when shear rate increases from 100 to 10 000 $s^{-1}$. A slight increase of viscosity is observed at shear rates higher than 2 000 $s^{-1}$. The control line solution is thus dilatant. Test line ink viscosity varies from 2.69 to 0.89 mPa s for the same shear rate ranges. The test line solution has a shear thinning behavior.

Equation 1 below is the expression of the shear rate as a function of gap and printing speed.

$$\textit{Equation 1:}\quad \dot{\gamma} = \frac{v}{h}\ ,$$

wherein $\dot{\gamma}$ is the shear rate ($s^{-1}$), $v$ is the velocity (m $s^{-1}$) and $h$ is the gap (m).

When shear rate varies from 100 to 10 000 $s^{-1}$, speed varies from 0.01 to 1 m $s^{-1}$ for a gap of 100 μm (1 x $10^{-4}$ m). Depending on ink viscosity and printing voltage, jetting speed thus varies from 0.1 to 25 m $s^{-1}$. Hence, high shear rates larger than 10 000 $s^{-1}$ and exceeding the rheometer measuring limits may be estimated.

Ideally, an inkjet printing ink must be Newtonian with a constant viscosity (1-10 mPa s) at varying shear rates. Though not Newtonian, biomolecule solutions are inkjet printable because of their low viscosities (< 2 mPa s).

Example 4: Surface chemical treatment analysis

The outer surface layers of paper substrates were analyzed by surface chemical analysis such as XPS and ATR-FTIR, thereby displaying the aforementioned bulk molecular structures.

XPS allows the identification of elements within 10 nm deep subsurface layers [48]. All papers are mainly composed of carbon and oxygen and therefore the XPS signal for these two elements is quite strong on every spectrum shown. Figure 8 displays O 1s orbital Binding Energy at 532 eV ± 0.35 eV, O 2s orbital Binding Energy at 24 eV ± 0.35 eV and C 1s orbital Binding Energy at 284 eV ± 0.35 eV) (Johansson, et Campbell, J. M. Reproducible XPS on biopolymers: cellulose studies. Surf. Interface Anal. 2004, 36, 1018-1022). Another peak at 405 ± 0.35 eV is noticeable onto nitrocellulose spectrum which is attributable to N 1s orbital.

According to its layout, ATR-FTIR allows the identification of chemical bonds within 2 μm deep subsurface

layers. All papers are mainly composed of a cellulosic backbone and therefore the IR signals for its typical bond vibrations are shared by every spectrum shown. Figure 9 displays these common bands attributable to O-H, C-H, C-C, C-O and O-C-O stretching vibrations. Besides, nitrocellulose manifests additional peaks (1638 ± 5 $cm^{-1}$ and 1275 ± 5 $cm^{-1}$) attributable to N-O stretching vibrations.

Example 5: analysis of Surface morphological structure

Beyond the chemical differences in molecular structure, the main difference between nitrocellulose and cellulose substrates lies in their surface physical structure. Thus, topological analysis was conducted in order to quantify the surface morphological structure by measuring its roughness (Ra). SEM imaging allowed visualizing surface morphology and microstructure of the unprinted substrates.

Line profiles of unprinted paper substrates (Figure 10) reveal that nitrocellulose surface is more homogeneous, smoother and has fewer and narrower pores compared to cellulose-based paper surfaces. Since profiles of the three cellulose-based papers were quite similar, only cellulose profile is displayed on Figure 10. Surface roughness (Ra) values (Figure 11) confirm that nitrocellulose is way smoother than cellulose-based papers. Pores size and arrangement pictured by SEM imaging (Figure 12) also corroborate the previous statements. SEM micrographs and roughness profiles predict that with the same ejected volume of antibodies, thicker and better resolution patterns will be printed on nitrocellulose. Thus, lower visual detection limits are expected to be reached with nitrocellulose membranes. This was supported by Määttanen *et al.* (Määttänen, A. et al., Colloids Surfaces A Physicochem. Eng. Asp. 2010, 367, 76-84) who demonstrated that wetting rate reduces with surface roughness increase. Besides, they explained that ink is quickly and completely absorbed into the depth of porous surfaces, thus leaving less ink deposit onto the substrate surface.

According to SEM imaging (Figure 12), glucose treatment seems to barely affect cellulose surface aspect. On the other hand, when paraffin treatment was performed, fewer pores were observed onto the surface. Regarding surface roughness (Figure 11), an increase was displayed by both glucose and paraffin treatments.

Example 6: surface chemical analysis of the printed substrates

After antibody had been printed onto the various paper substrates, their outer surface layers were analyzed anew in order to detect any change stemming from the biomolecules. The XPS signal from carbon and oxygen is still quite strong on every spectrum shown (Figure 13). Additional peaks at 397.5 ± 0.35 eV have come out onto all the spectra which are attributable to N 1s orbital from antibody molecules. Since spectra of the three cellulose-based papers were quite similar, only cellulose spectrum is displayed on Figure 13.

With regard to IR analysis, the intense spectra from initial substrates hid most of the characteristic bands pointing out the immobilized antibodies (Figure 14). Therefore, the amide bands specific to proteins are barely perceivable. Only amide II at 1547 ± 5 $cm^{-1}$ could be clearly identified onto nitrocellulose substrates.

*Surface morphological structure*

After antibody had been printed onto the various paper substrates, their surface morphology and microstructure were visualized anew (not shown) by SEM imaging in order to detect any change stemming from the biomolecules. Unfortunately, the microscope resolution was not high enough to enable a direct visualization of antibody deposit. However, a thin new layer seems to have appeared on cellulose-based substrates when comparing to Figure 12.

Lateral flow immunoassays (LFIAs)

Antibody solutions were printed onto the raw (nitrocellulose and cellulose) and pretreated (glucose-cellulose and paraffin-cellulose) substrates. 5 layers were printed in order to get strong color intensity. Antibodies were then adsorbed onto nitrocellulose substrate and photoimmobilized onto cellulose substrates (cellulose, glucose-cellulose and paraffin-cellulose). Lateral flow immunoassays (LFIAs) evaluated the biological activity of the printed antibodies and the visual detection limit of the various bioactive membranes, thereby allowing characterization of the various substrates in terms of biosensing performances. First, the immobilization ability of the various membranes was confirmed by revelation with gold-labeled goat anti-mouse tracer (see control strips in Figure 15). Then, their biological activity was assessed by exposition to OVA antigen and revealed by gold-labeled murine anti-OVA tracer (sandwich immunoassay) (see OVA strips in Figure 15). Each test was performed in triplicate.

The first fact to notice is that though antibodies were barely perceivable with the various surface analysis performed (XPS, IR or SEM), they are well visible after either revelation with goat anti-mouse tracer (control strips) or bioactivity assessing immunosandwich (OVA strips). With regard to biological activity, few aforementioned results (see above) remain. Dilutive effect is still clearly perceptible. There is still a false positive result with nitrocellulose that compels

to appraise its VDL at 5 ng $mL^{-1}$ (Figure 15a). The other VDLs are 50 ng $mL^{-1}$ for cellulose (Figure 15b), 10 to 25 ng $mL^{-1}$ for glucose-cellulose (Figure 15c), and 25 to 50 ng $mL^{-1}$ for paraffin cellulose (Figure 15d). While nitrocellulose's VDL is still the same as described above, cellulose's VDL is now higher. Since all test lines coloring seems weaker than in Figure 4c, this inter-assay variability could originate from tracer variability due to the use of another batch of colloidal gold. On another hand, the intra-assay comparison of the different substrates reveals that both glucose and paraffin enrichment slightly improved cellulose performances although they are still lower than nitrocellulose's. Besides, glucose-cellulose appeared to be the most sensitive cellulose-based substrate. This could be explained by a slight decrease in surface porosity, as expected.

Example 7: Inkjet printing of complex designs

As previously mentioned, one advantage of inkjet printing dispensing method is the freedom in design of the printed pattern. This advantage was illustrated here by printing antibodies according to their nature and function, thereby making the user manual not so useful anymore. Since bottom line was dedicated to capture OVA antigen, murine anti-OVA monoclonal antibodies printing drew the abbreviation OVA. Similarly, anti-mouse antibodies were printed on the top line according CTRL abbreviation as the top line aimed to control the smooth progress of the immunoassay. After antibody solutions had been dispensed onto the substrates (1-layer inkjet printing), the antibodies were either adsorbed onto nitrocellulose or photoimmobilized onto cellulose. Their biological activity was put to the test by exposition to OVA antigen (500 ng $mL^{-1}$) and simultaneously revealed by gold-labeled murine anti-OVA tracer (Figure 16). Colors observed, along with their intensities, were consistent with previous results (see above). Finally, as expected, the drawn patterns allowed direct reading of the test results. This process therefore enables to doubly check the nature of the target antigen (on the box and on the strip), thereby avoiding ambiguousness when box label is partly erased. Firstly, this can permit to save valuable assay devices in remote areas in the developing world. In addition, this double-check can be a huge asset in developed countries in emergency situations, in emergency rooms or in military settings, where the result of the assay impacts on people's lives.

The herein exemplified method thus constitutes a fast, simple, cost-saving and environmentally friendly method for strong and precisely localized immobilization of antibodies onto paper has been described herein. Further, the combination of inkjet printing of biomolecules with a chemical-free photografting procedure according to the invention together enable to easily, rapidly and permanently immobilize antibodies onto cellulose-based papers according to any pattern desired. The inkjet printing dispensing method has the great advantage of saving the expensive biomolecules. The photografting procedure has the one of being harmless to chemical-sensitive biomolecules. Cellulose pretreated sheets have been studied, and demonstrated to result in membranes challenging nitrocellulose performances. Cellulose performances appeared slightly lower than nitrocellulose's though. But this phenomenon probably stemmed from the physical differences, such as surface porosity variation, between nitrocellulose and cellulose substrates.

The photografting of pretreated cellulose, and more generally of substantially non-porous carriers meets the need for sensing devices development to rapidly, robustly and abundantly immobilize biomolecules onto substrates or carriers according to complex patterns and at low cost. The expounded process thus provides a powerful tool for immobilizing chemical-sensitive proteins according to complex patterns and onto various substantially non-porous carriers, including cellulose-based paper sheets.

Further, several additional filling substances have been tested on cellulose sheets:

- Poly(acrylic acid) sodium salt, 35% in $H_20$ Mw 60.000 (PolySciences Inc);
- Poly(acrylic acid) sodium salt, 35% in $H_20$ Mw 250.000 (Sigma Aldrich);
- Poly(acrylic acid) powder Mw 1.000.000 (PolySciences Inc);
- Poly(styrenesulfonic acid-Maleic anhydride), 3 :1, Low Mn (Polysciences Inc);
- Poly(vinylphosphonic acid), 30% in $H_20$;
- PolyStyrene solid, Mw 192.000 (Sigma Aldrich); and
- Poly(ethyleneglycol) Mw 950-1.050 (Sigma Aldrich).

Example 8: pretreatment of cellulose sheets with a solution of Poly(acrylic acid) sodium salt 35% (Mw 60.000)

A 35% w/v solution of Poly(acrylic acid) sodium salt (Mw 60.000 (PolySciences Inc)) was solubilized in $H_20$ and filtered as disclosed above on XEROX Premier® or on Whatman CF1® cellulose sheets. Filtering was stopped when the polymer solution became unable to flow through the cellulose sheet. The resulting sheets were then dried at room temperature for 48 hours.

Example 9: pretreatment of cellulose sheets with a solution of Poly(acrylic acid) sodium salt 35% (Mw 250.000)

A 35% w/v solution of Poly(acrylic acid) sodium salt (250.000 (Sigma Aldrich)) was solubilized in $H_20$ and filtered as disclosed above on XEROX Premier® or on Whatman CF1® cellulose sheets. Filtering was stopped when the polymer solution became unable to flow through the cellulose sheet. The resulting sheets were then dried at room temperature for 48 hours.

The porosity of the treated cellulose sheets was analyzed by Scanning Electron Microscopy (SEM). Results are presented in Figure 17A (untreated CF1 sheet), and 17B) (CF1 sheet obtained after treatment).

Example 10: pretreatment of cellulose sheets with a solution of PolyStyrene

A solution of PolyStyrene (Mw 192.000 (Sigma Aldrich)) was prepared by dissolving 3g of polystyrene in 10 mL pure acetone (Sigma Aldrich). The supernatant only was collected, then filtered on XEROX Premier® or on Whatman CF1® cellulose sheets. Filtering was stopped when the polymer solution became unable to flow through the cellulose sheet. The resulting sheets were then dried at room temperature for 48 hours.

Example 11: pretreatment of cellulose sheets with a solution of Poly(acrylic acid) (Mw 1.000.000)

A solution of Poly(acrylic acid) (Mw 1.000.000 (PolySciences Inc)) was prepared by dissolving 200mg of polyacrylic acid in 10 mL Milli-Q $H_20$. The solution was then filtered on XEROX Premier® or on Whatman CF1® cellulose sheets. Filtering was stopped when the polymer solution became unable to flow through the cellulose sheet. The resulting sheets were then dried at room temperature for 48 hours.

Example 12: pretreatment of cellulose sheets with a solution of Poly(acrylic acid) (Mw 250.000), Poly(vinylphosphonic acid), Poly(styrenesulfonic acid-Maleic anhydride, and Low Mn

A solution containing i) 2 mL of a 35% w/v Poly(acrylic acid) sodium salt solution (Mw 250.000 (Sigma Aldrich)) in $H_20$, ii) 2 mL of a 30% Poly(vinylphosphonic acid) solution, in $H_20$, and iii) 15 mL of Milli-Q $H_20$ containing 1g of Poly(styrenesulfonic acid-Maleic anhydride), Low Mn (Polysciences Inc) 3:1, and 1,5g of Poly(ethyleneglycol) Mw 950-1.050 (Sigma Aldrich) was filtered on XEROX Premier® or on Whatman CF1® cellulose sheets. Filtering was stopped when the polymer solution became unable to flow through the cellulose sheet. The resulting sheets were then dried at room temperature for 48 hours.

Example 13:pretreatment of cellulose sheets with a solution of Poly(acrylic acid) (Mw 250.000) then by a solution of PolyStyrene

A 35% solution of Poly(acrylic acid) sodium salt (Mw 250.000 (Sigma Aldrich)) in $H_20$ was filtered on XEROX Premier® or on Whatman CF1® cellulose sheets. The resulting sheets were then dried at room temperature for 24 hours.

A solution of PolyStyrene (Mw 192.000 (Sigma Aldrich)) was prepared by dissolving 3g of polystyrene in 10 mL pure acetone (Sigma Aldrich). The supernatant only was collected, then filtered on the dried cellulose sheets. Filtering was stopped when the polymer solution became unable to flow through the cellulose sheet. The resulting sheets were then dried at room temperature for 48 hours.

Example 14: pretreatment of cellulose sheets with a solution of Poly(acrylic acid) (Mw 250.000) then by a solution of PolyStyrene

A 35% solution of Poly(acrylic acid) sodium salt (Mw 250.000 (Sigma Aldrich)) in $H_20$ was filtered as described previously on XEROX Premier® or on Whatman CF1® cellulose sheets. Filtering was stopped and the cellulose sheets were dried at room temperature for 48 hours. The supernatant of a solution of PolyStyrene (Mw 192.000 (Sigma Aldrich)) prepared by dissolving 3g of polystyrene in 10 mL pure toluene (Sigma Aldrich) was then filtered on the same cellulose sheets. Filtering was stopped when the polymer solution became unable to flow through the cellulose sheet. The resulting sheets were then dried at 40 °C for 48 hours.

Example 15: Photochemical grafting of proteins on polyethyle terephthalate (PET)

A solution of monoclonal antibody anti-OVA, at a concentration of 1 mg/ml, was printed onto a support of polyethylene terephthalate (PET) of a thickness of 100 µm, with an inkjet printer Dimatix Material Printer DMP-2800,

having a distance printing head/support of 0.75 mm. The printed support was dried in an oven, at standard atmospheric pressure, at 37-40°C, then irradiations were conducted at room temperature under a 365 nm light, for 15 minutes. The grafted surface was then subjected to washing with ultrasound, then observed by SEM imaging. Results are displayed on Figure 18A) and B). As could be seen in figure 18, trace amounts of proteins can still be observed after washing with ultrasound. Further, IR analysis of the grafted PET surface was conducted before and after washing with water and ultrasound. Results are displayed in Figure 19. Peaks corresponding to primary amines, $-NH_2$ (around 1660 $cm^{-1}$), and to secondary amines, -NH (around 1550 $cm^{-1}$), can still be observed even after washing with water and ultrasound.

## Claims

**1.** A process for immobilizing biomolecules on a substantially non-porous carrier comprising the steps of:

(i) impregnating said carrier with a solution containing the said biomolecules; and
(ii) irradiating the impregnated carrier resulting from step (i) with a light of a wavelength of at least 340 nm;
wherein said carrier is composed of a naturally substantially non-porous material such as for example polyethylene terephthalate (PET), Polyethylene naphthalate (PEN), Poly(methyl methacrylate) (PMMA), Polyurethane (PU), Poly(vinyl chloride) (PVC), Polyethylene (PE), Polystyrene (PS), Polylactate, polyamide, and combinations thereof, or of a porous material that has been rendered substantially non-porous; and wherein said biomolecules are not functionalized.

**2.** The process according to claim **1,** further comprising a step of drying said impregnated carrier after step (i) and before step (ii).

**3.** The process according to any one of claims **1** or **2,** further comprising at least one step of washing the irradiated carrier resulting from step (ii).

**4.** The process according to any one of claims **1** to **3,** wherein the irradiation light has a wavelength of from 340 nm to 800 nm, preferably of from 340 nm to 400 nm, preferably of from 400 nm to 800 nm, and is preferably of about 365 nm.

**5.** The process according to any one of claims **1** to **4,** wherein the irradiation step (ii) is performed with a photoenergy of from 1 $mJ/cm^2$ to 500 $J/cm^2$, preferably of from 1 $J/cm^2$ to 80 $J/cm^2$, and is preferably of about 10 $J/cm^2$.

**6.** The process according to any one of claims **1** to **5,** wherein said naturally substantially non-porous material is selected from the group consisting of polyethylene terephthalate (PET), Polyethylene naphthalate (PEN), Poly(methyl methacrylate) (PMMA), Polyurethane (PU), Poly(vinyl chloride) (PVC), Polyethylene (PE), Polystyrene (PS), Polylactate, polyamide, and combinations thereof.

**7.** The process according to any one of claims **1** to **5,** further comprising a preliminary step, before step i), of preparing a substantially non-porous carrier starting from a porous material, comprising the steps of:

a) impregnating said porous material with at least one filler until saturation thereof; and
b) drying the impregnated material resulting from a).

**8.** The process according to claim **7,** wherein said porous material is cellulose.

**9.** The process according to any one of claims **7** or **8,** wherein said at least one filler is selected in the group consisting of glucose, paraffin, sulfonated polymers, polyacrylic acid (PAA), poly-2-hydroxyethyl methacrylate (PHEMA), polymethyl methacrylate (PMMA), poly(poly(ethylene glycol) dimethacrylate) (PPEGDMA), polypropylene (PP), poly(styrene sulfonic acid - Maleic anhydride), poly(vinyl phosphonic acid), polyethyleneglycol, as well as salts thereof and/or combinations thereof.

**10.** The process according to any one of claims **1** to **9,** wherein said biomolecule is selected from the group consisting of proteins or peptides, such as antibodies, antigens, enzymes, transcription factors, protein domains or binding proteins.

**11.** A grafted carrier obtained by the process of any one of claims **1** to **10,** wherein said carrier comprises biomolecules

immobilized thereonto.

**12.** A bioassay device comprising at least one carrier according to claim **11** wherein said bioassay device is preferably an immunoassay device such as an immunochromatographic strip or an immunochromatographic multiplex system.

**13.** Use of at least one carrier according to claim **11** or of a bioassay device according to claim **12** for diagnosis, affinity chromatography, proteomics, genomics and/or drug screening.

**14.** A bioassay kit comprising at least one carrier according to claim **11** or at least one bioassay device according to claim **12.**

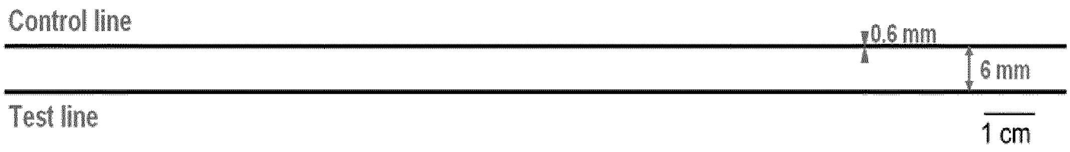


FIG. 1

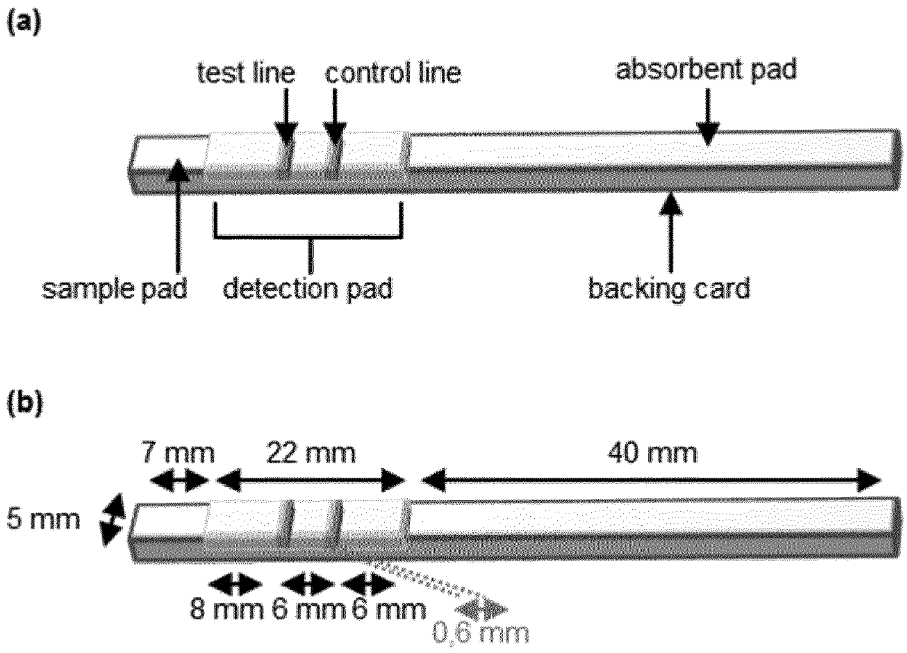


FIG. 2

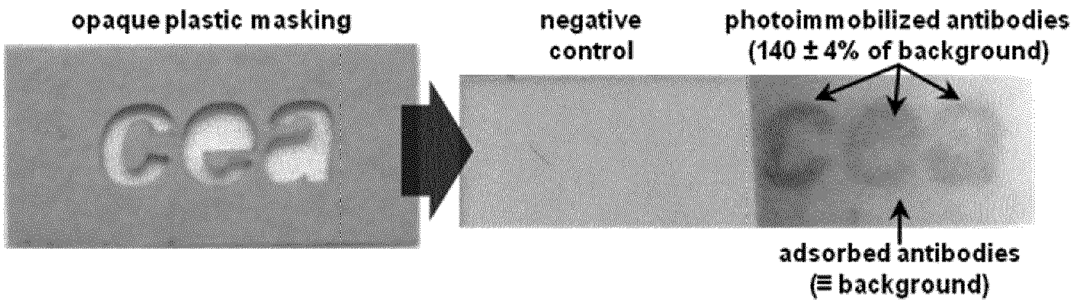


FIG. 3

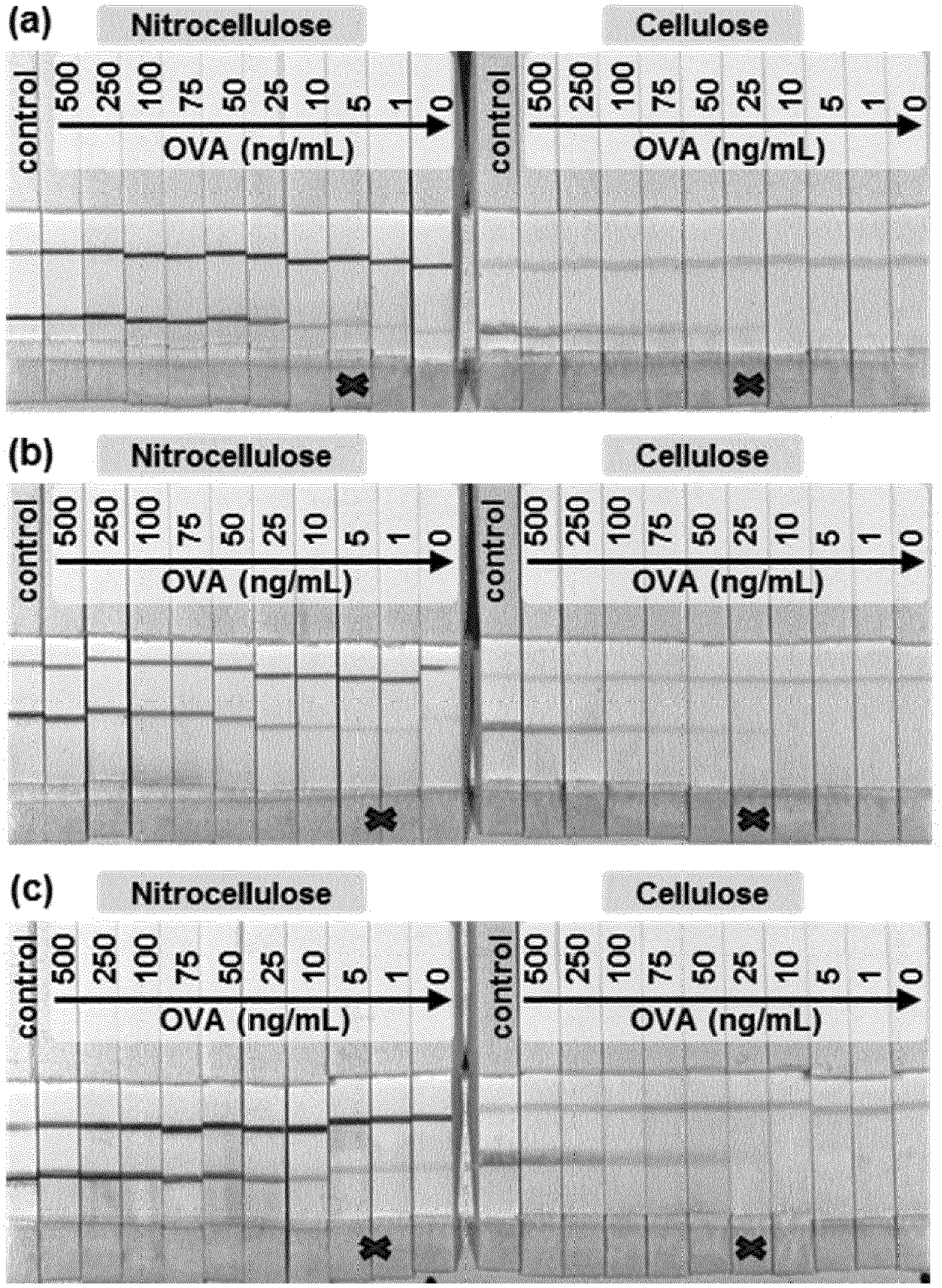
(a)
Nitrocellulose
Cellulose
control
500
250
100
75
50
25
10
5
1
0
OVA (ng/mL)
(b)
Nitrocellulose
Cellulose
control
500
250
100
75
50
25
10
5
1
0
OVA (ng/mL)
(c)
Nitrocellulose
Cellulose
control
500
250
100
75
50
25
10
5
1
0
OVA (ng/mL)

FIG. 4

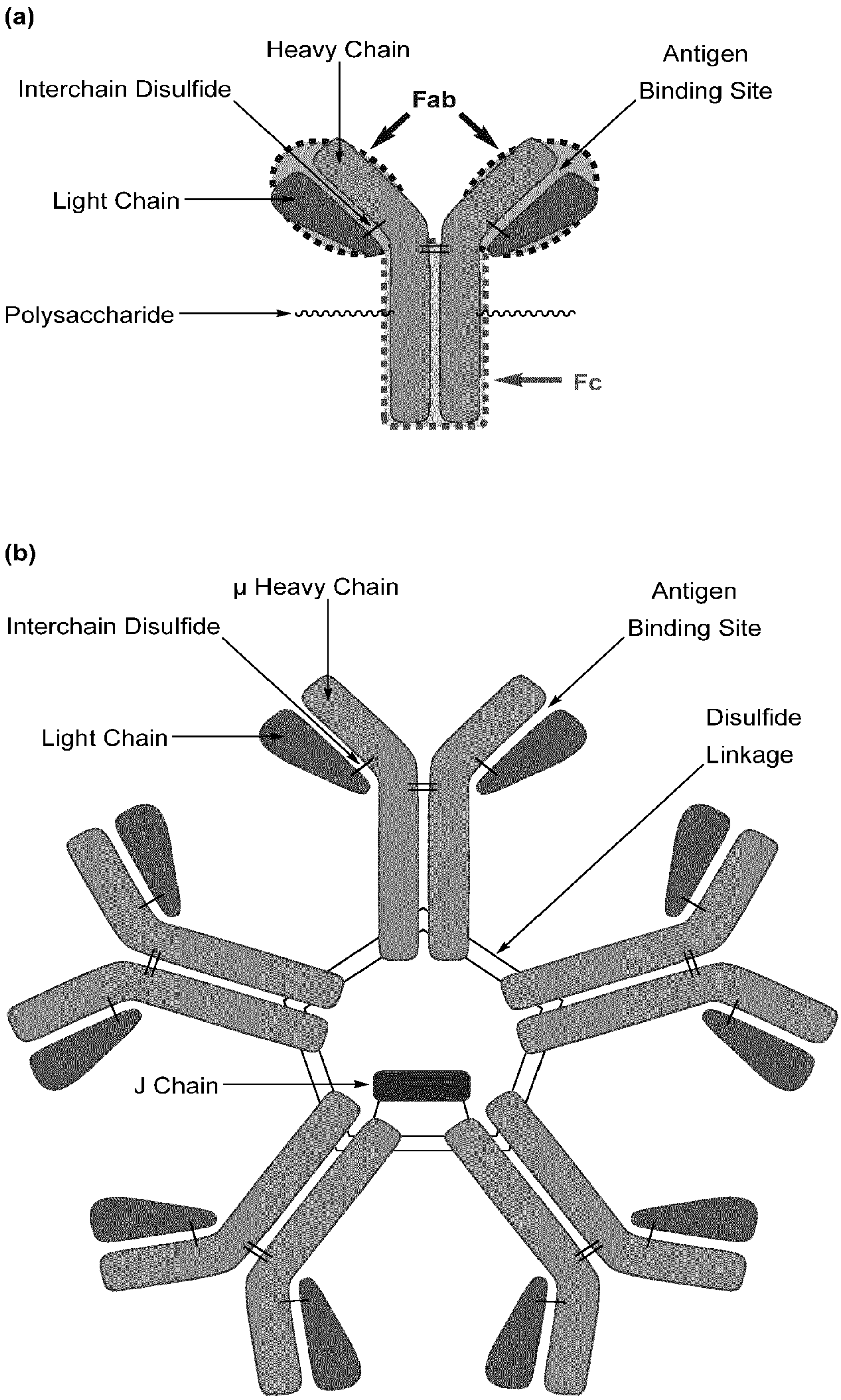
(a)
Heavy Chain
Antigen
Binding Site
Interchain Disulfide
Fab
Light Chain
Polysaccharide
Fc
(b)
μ Heavy Chain
Antigen
Binding Site
Interchain Disulfide
Light Chain
Disulfide
Linkage
J Chain

FIG. 5

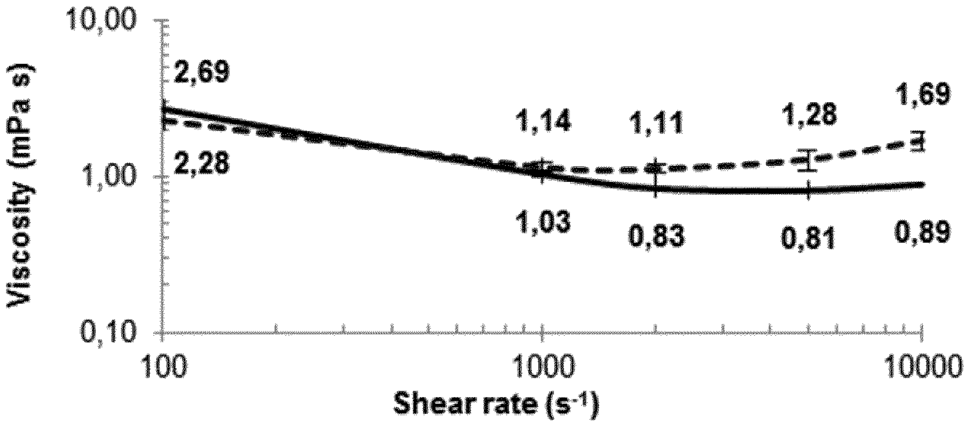


**FIG. 6**

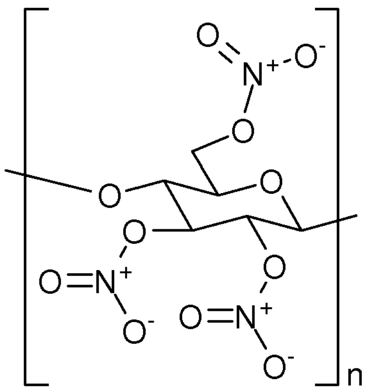

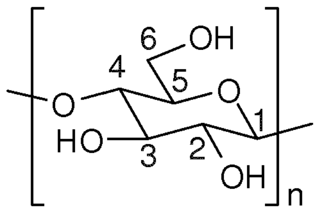

(a) nitrocellulose cellulose

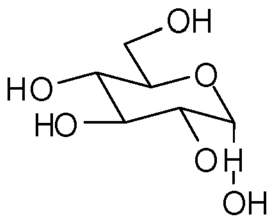

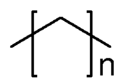

20 < n < 40

(b) D-(+)-glucose paraffin

**FIG. 7**

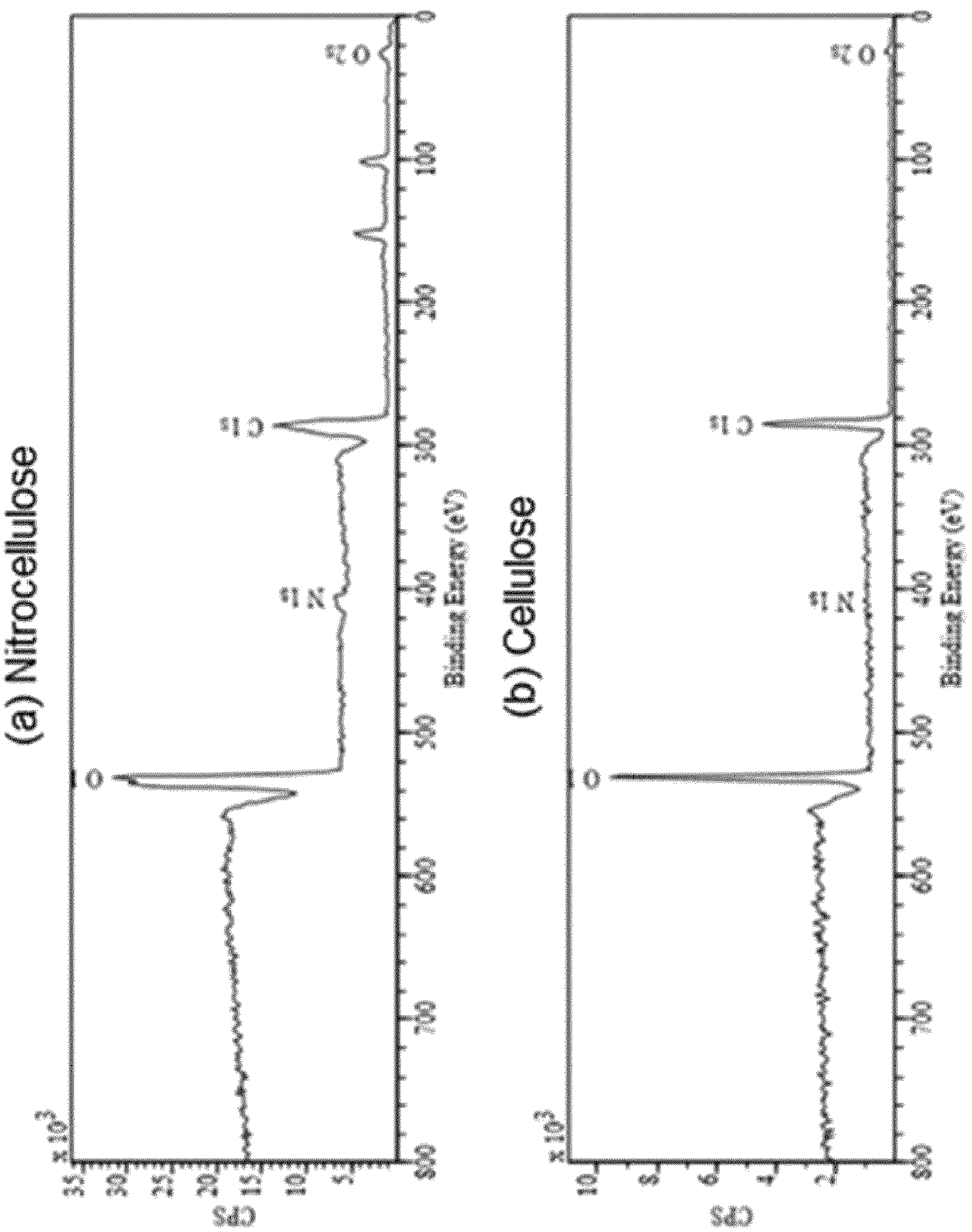
(a) Nitrocellulose
(b) Cellulose
Binding Energy (eV)
CPS
x 10$^3$
O 1s
N 1s
C 1s
O 2s

FIG. 8A-B

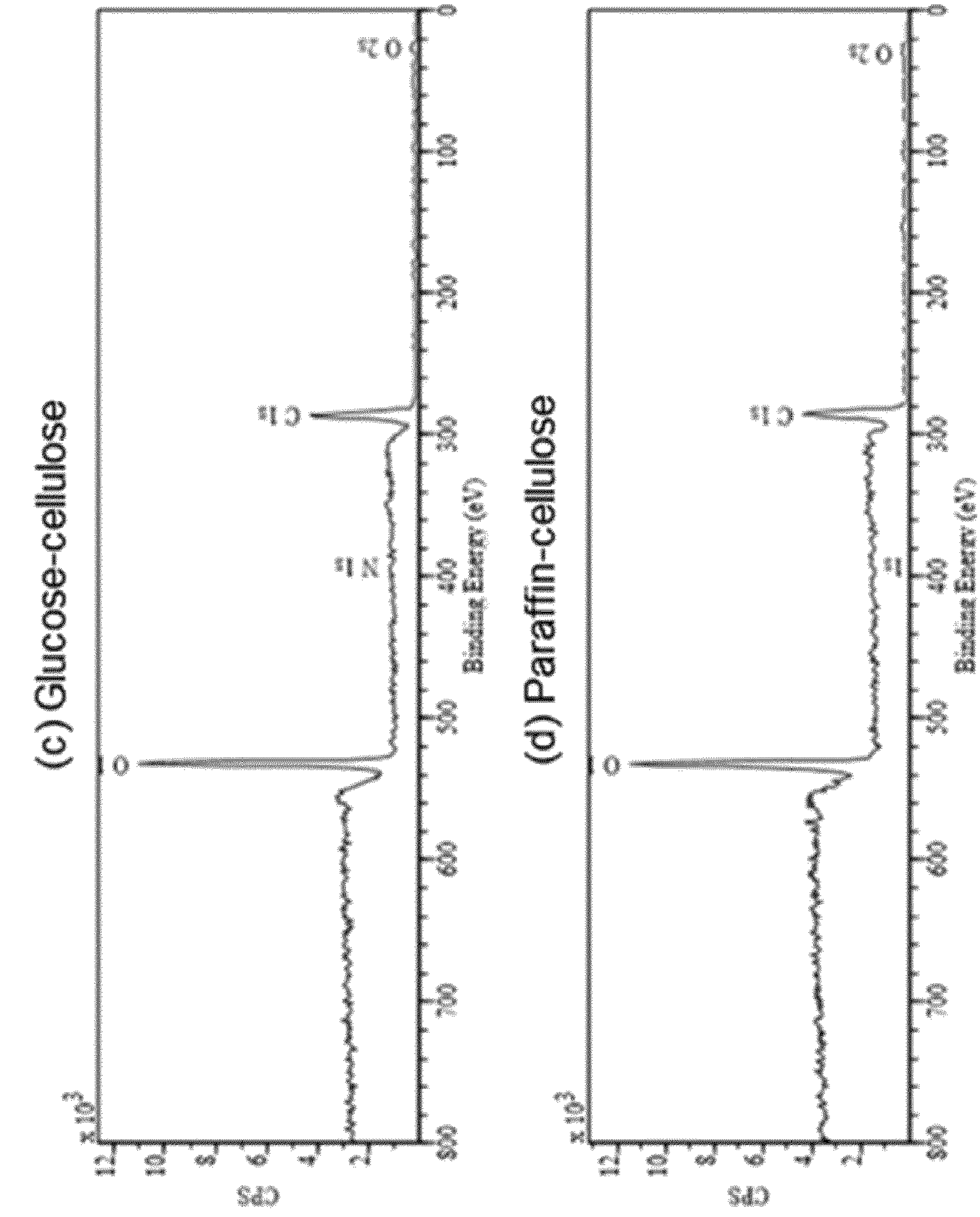
(c) Glucose-cellulose
(d) Paraffin-cellulose
CPS
x 10^3
Binding Energy (eV)
O 1s
N 1s
C 1s
O 2s

FIG. 8C-D

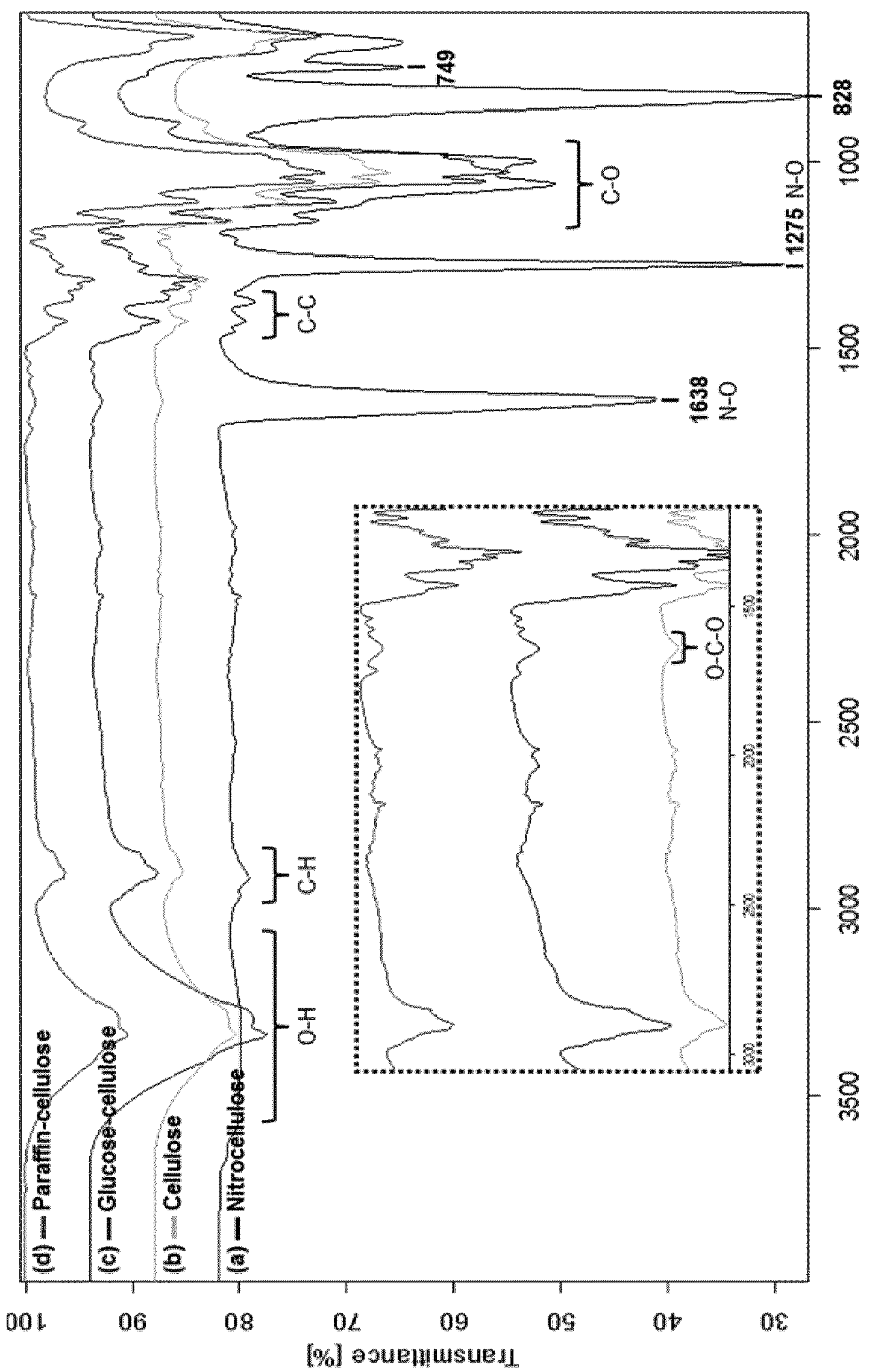
(d) — Paraffin-cellulose
(c) — Glucose-cellulose
(b) — Cellulose
(a) — Nitrocellulose
O-H
C-H
C-C
C-O
O-C-O
1638
N-O
1275 N-O
828
749
Transmittance [%]
Wavenumber cm-1
100
90
80
70
60
50
40
30
3500
3000
2500
2000
1500
1000

FIG. 9

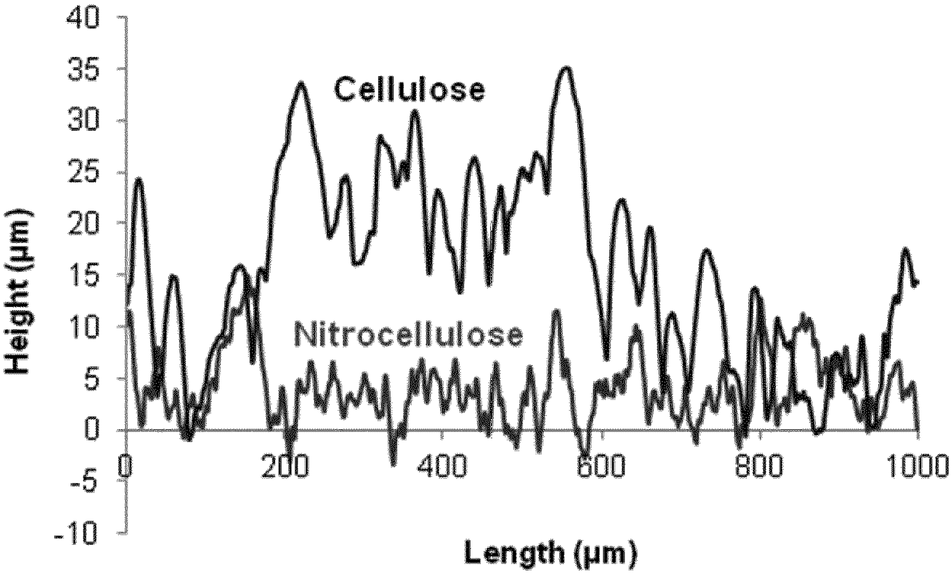
Height (µm)
Length (µm)
Cellulose
Nitrocellulose
40
35
30
25
20
15
10
5
0
-5
-10
0
200
400
600
800
1000

FIG. 10

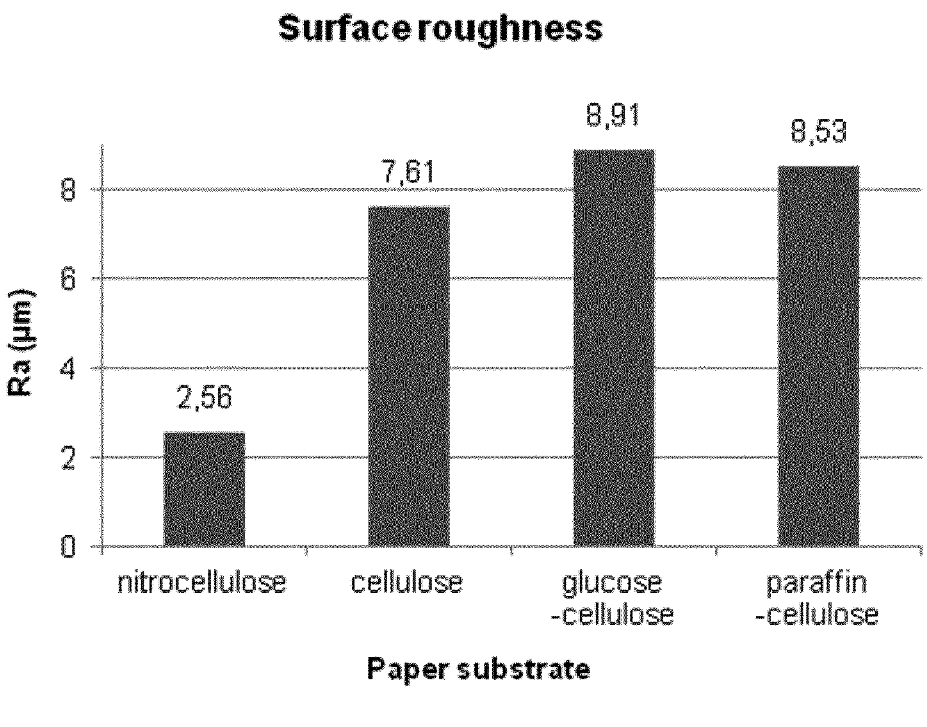
Surface roughness
Ra (µm)
Paper substrate
8,91
8,53
7,61
2,56
8
6
4
2
0
nitrocellulose
cellulose
glucose -cellulose
paraffin -cellulose

FIG. 11

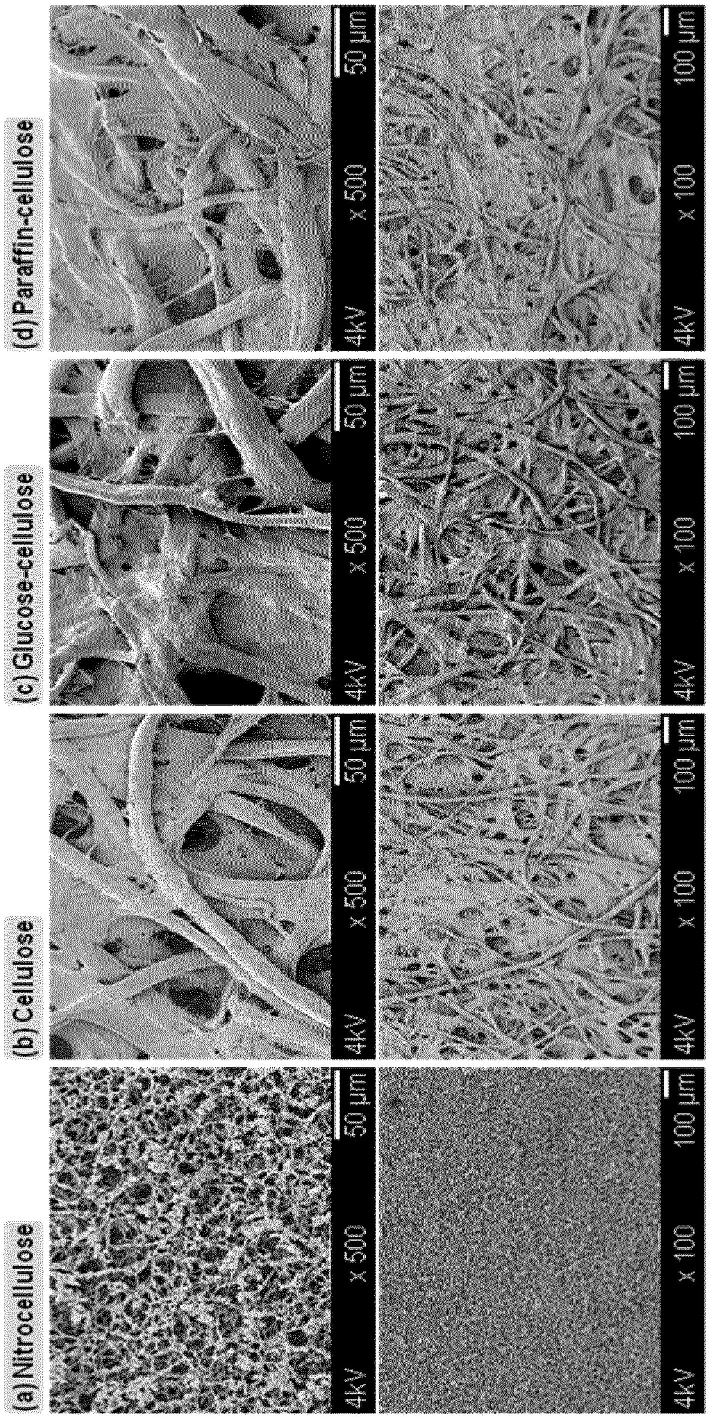
(a) Nitrocellulose
(b) Cellulose
(c) Glucose-cellulose
(d) Paraffin-cellulose
4kV x 500 50 µm
4kV x 100 100 µm

FIG. 12

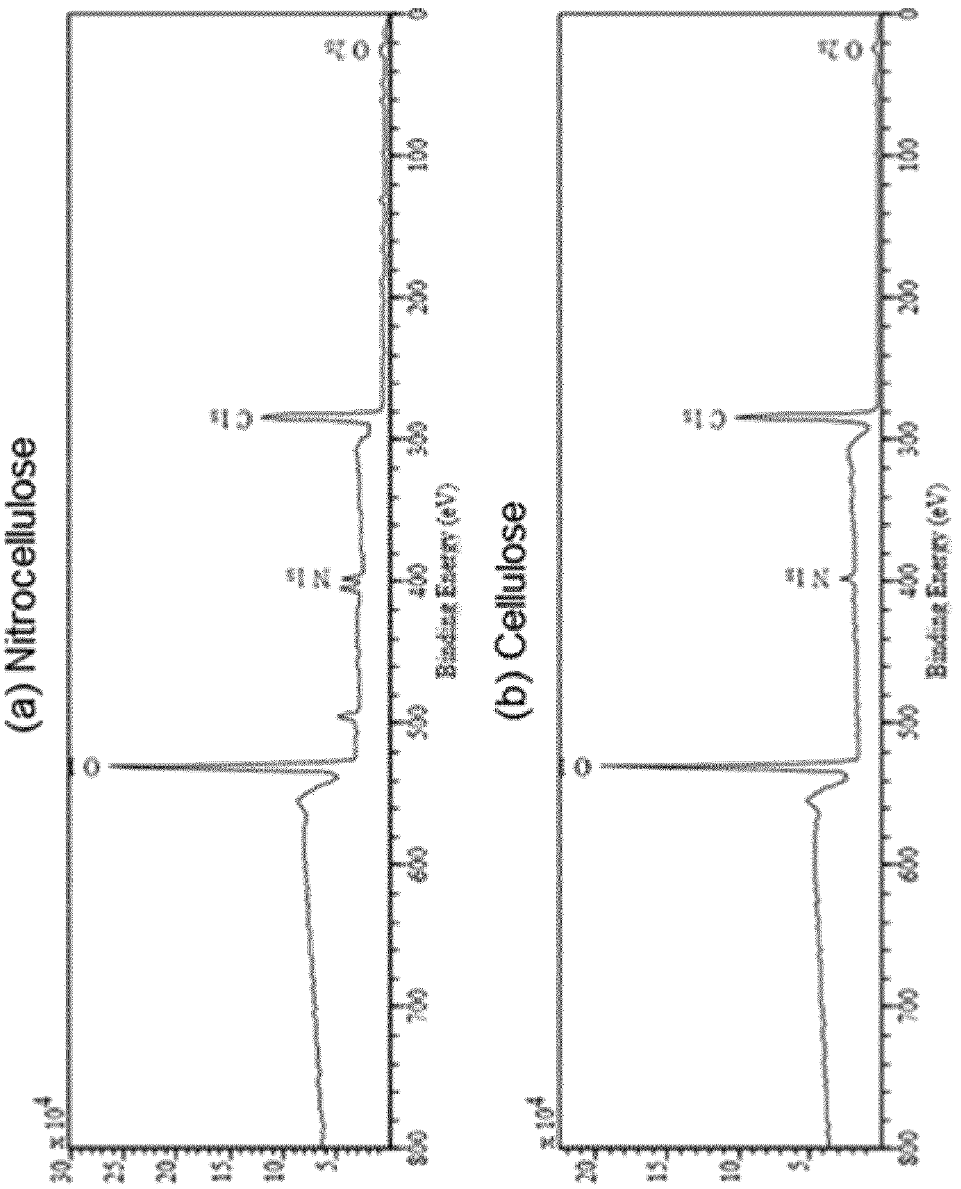
(a) Nitrocellulose
(b) Cellulose
CPS
x 10^4
Binding Energy (eV)
O 1s
N 1s
C 1s
O 2s

**FIG. 13**

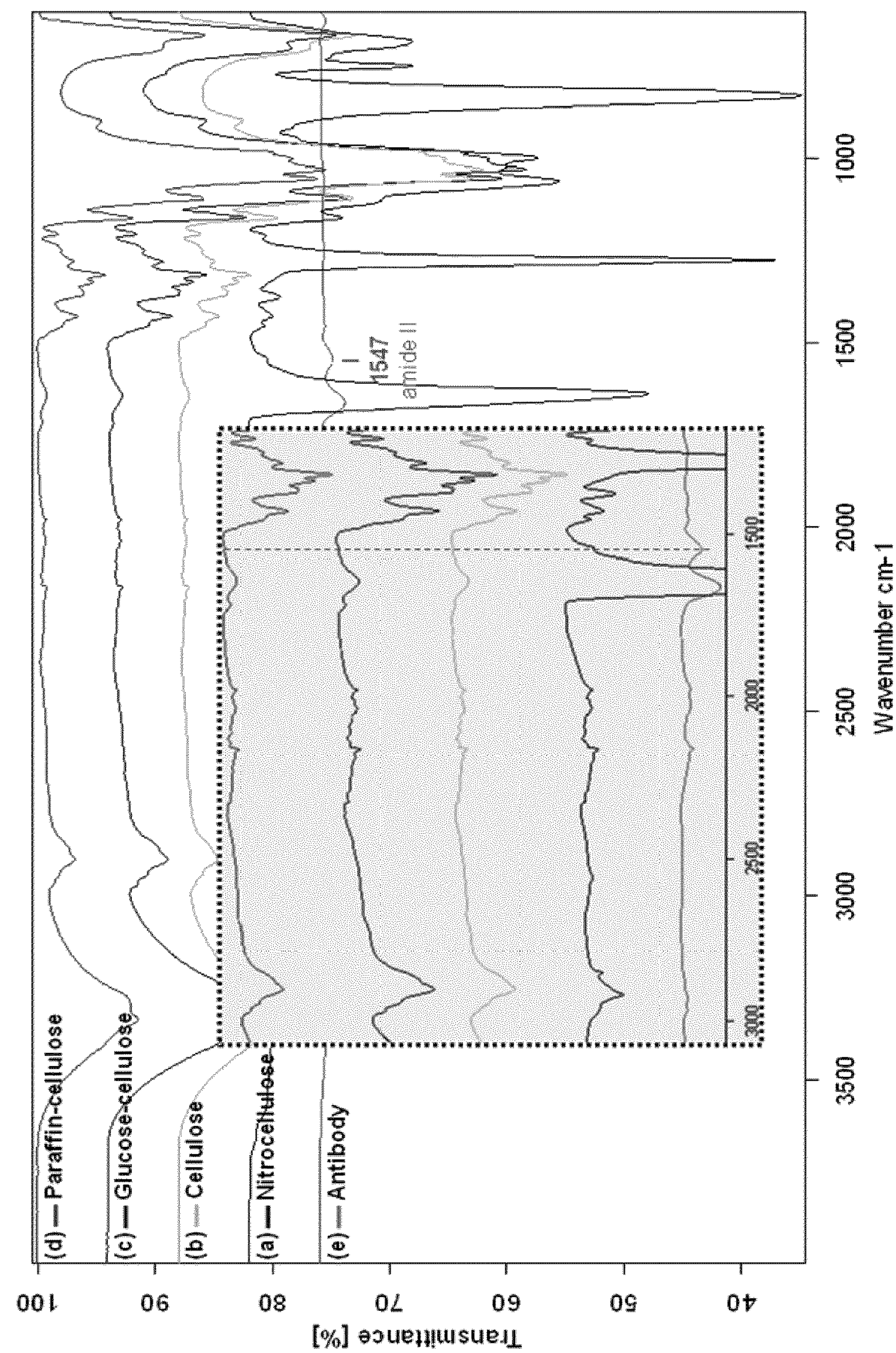
(d) — Paraffin-cellulose
(c) — Glucose-cellulose
(b) — Cellulose
(a) — Nitrocellulose
(e) — Antibody
1547
amide II
Transmittance [%]
Wavenumber cm-1
100
90
80
70
60
50
40
3500
3000
2500
2000
1500
1000

FIG. 14

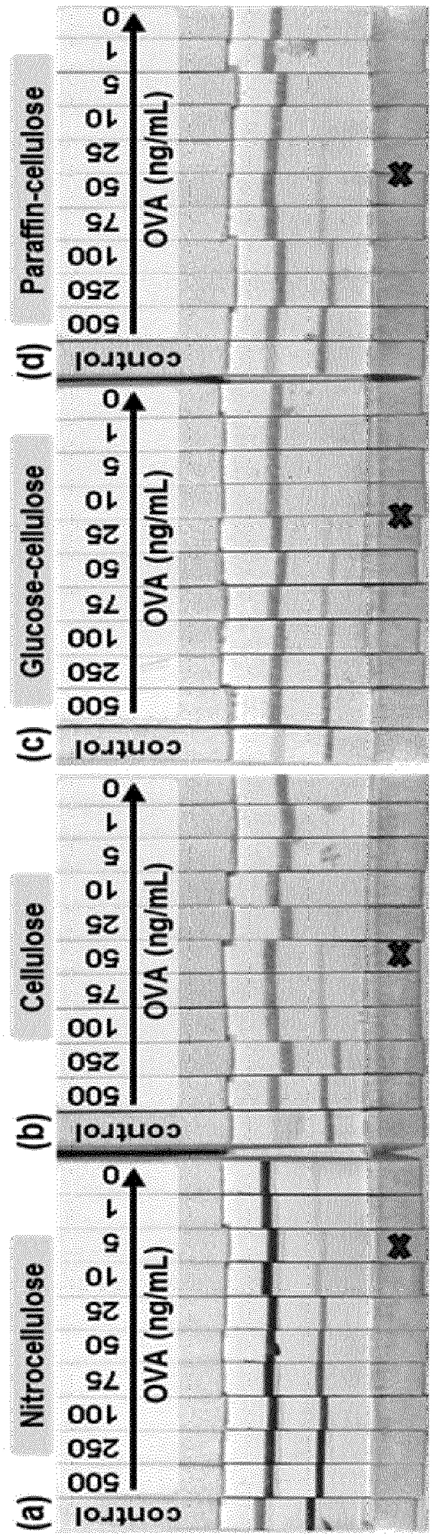
(a) Nitrocellulose
(b) Cellulose
(c) Glucose-cellulose
(d) Paraffin-cellulose
OVA (ng/mL)
control
500
250
100
75
50
25
10
5
1
0

FIG. 15

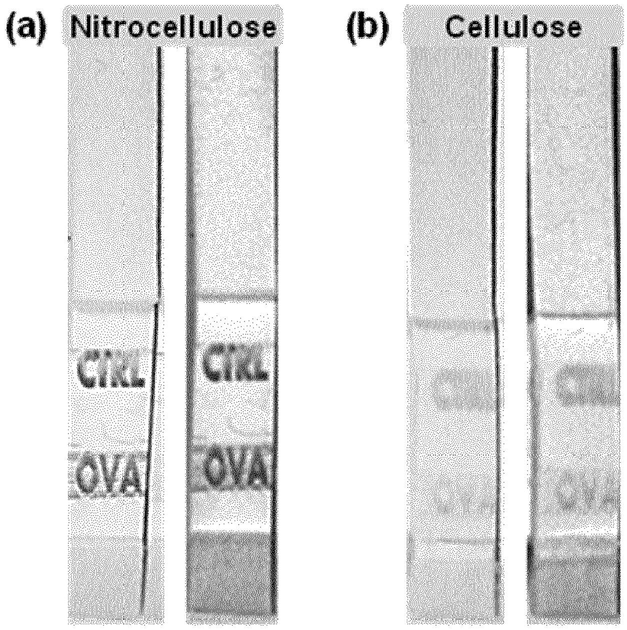


FIG. 16

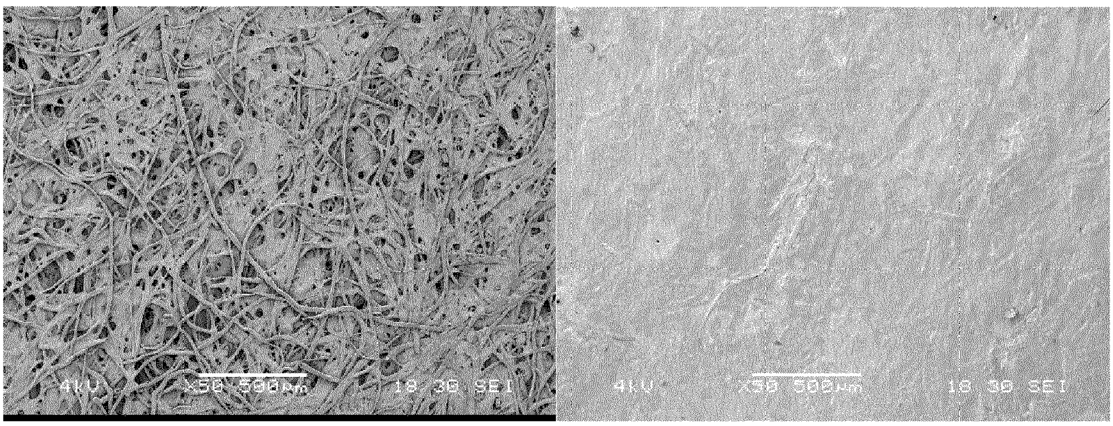


A) B)

FIG. 17

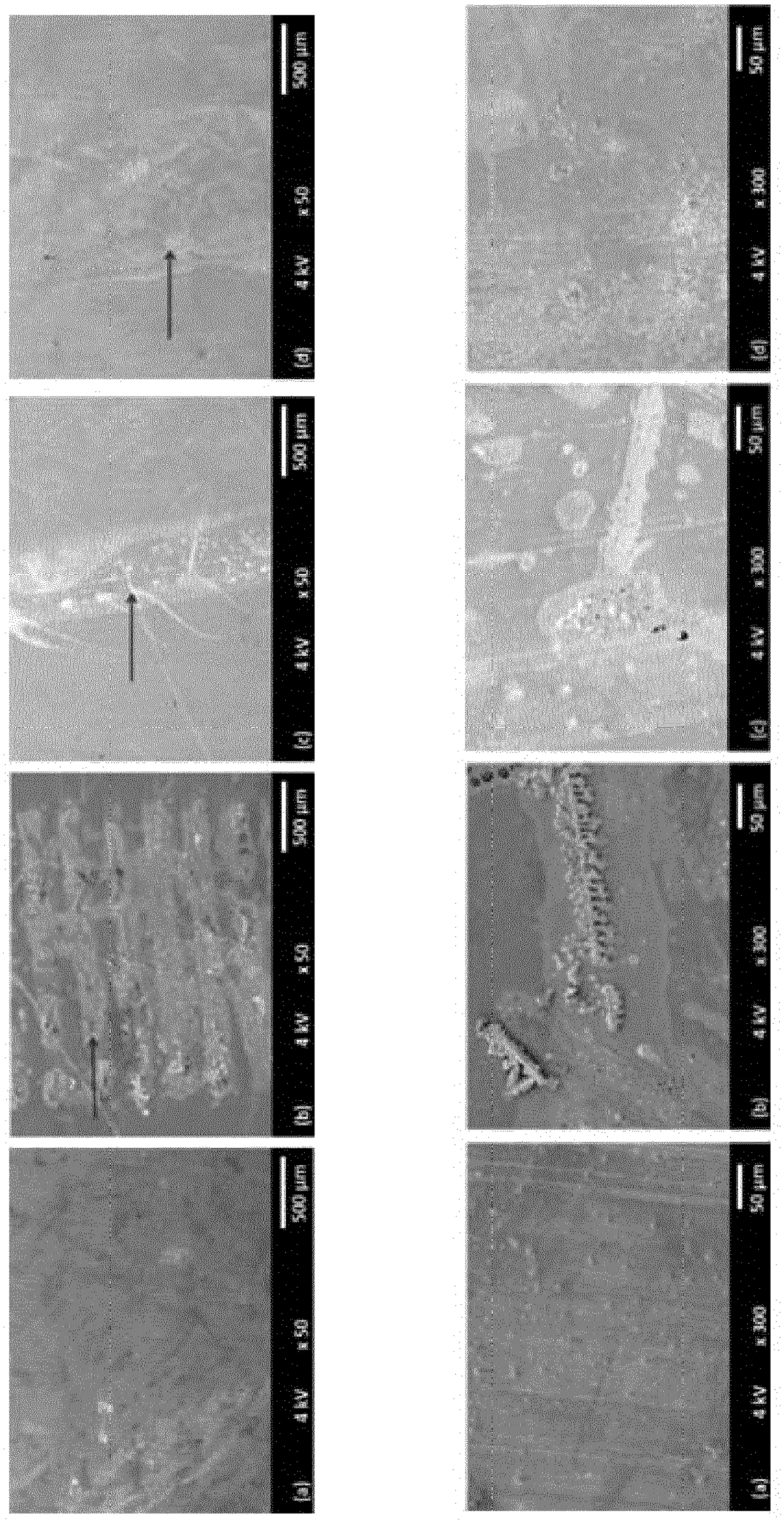
(d) 4 kV x 50 500 µm
(c) 4 kV x 50 500 µm
(b) 4 kV x 50 500 µm
(a) 4 kV x 50 500 µm
(d) 4 kV x 300 50 µm
(c) 4 kV x 300 50 µm
(b) 4 kV x 300 50 µm
(a) 4 kV x 300 50 µm

A)

B)

FIG. 18

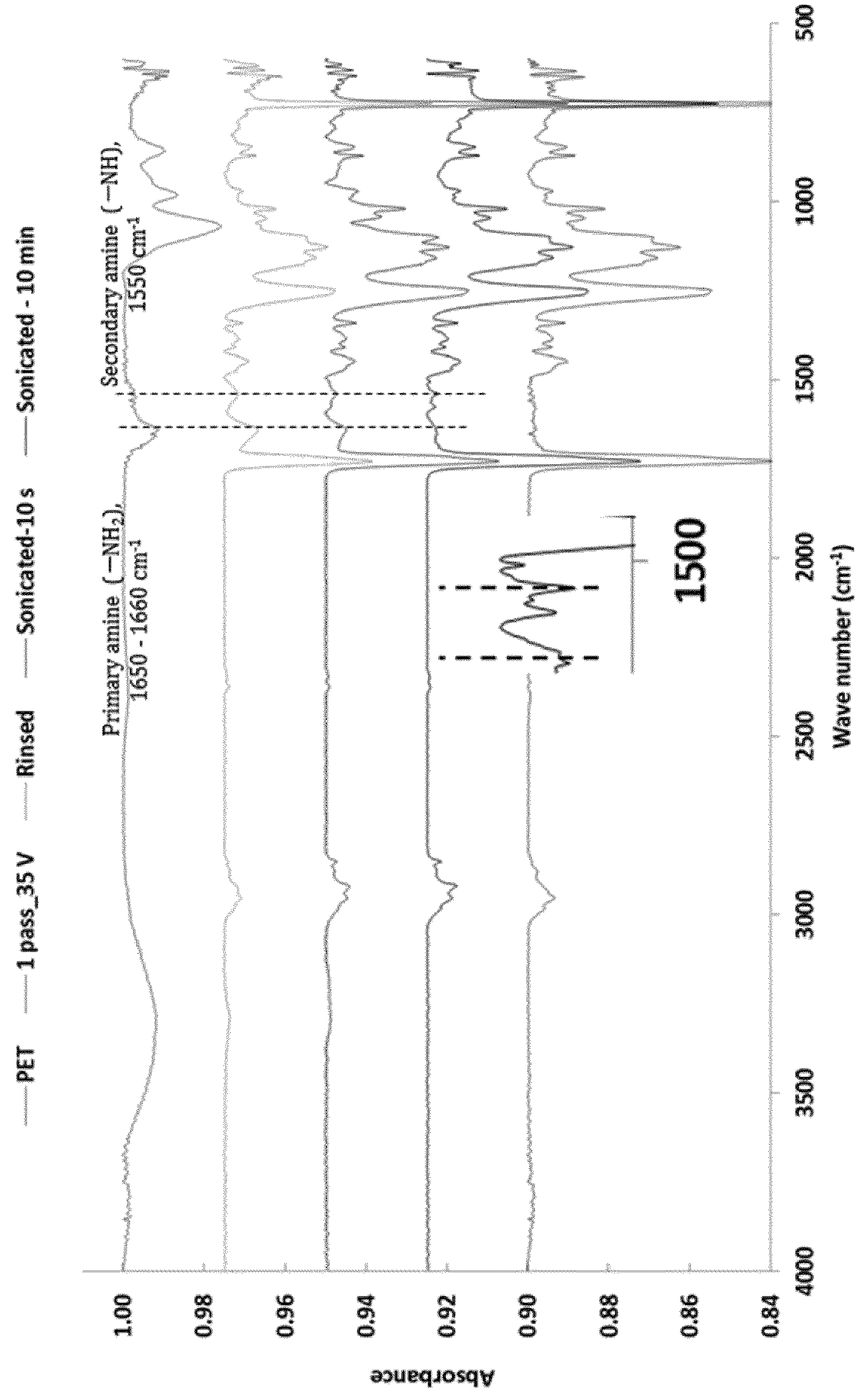
PET
1 pass_35 V
Rinsed
Sonicated-10 s
Sonicated - 10 min
Primary amine (—$NH_2$),
1650 - 1660 $cm^{-1}$
Secondary amine (—NH),
1550 $cm^{-1}$
1500
Absorbance
1.00
0.98
0.96
0.94
0.92
0.90
0.88
0.86
0.84
4000
3500
3000
2500
2000
1500
1000
500
Wave number ($cm^{-1}$)

FIG. 19

Europäisches Patentamt
European Patent Office
Office européen des brevets

# EUROPEAN SEARCH REPORT

Application Number
EP 14 18 0423

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/115558 A1 (KIM WAN JOONG [KR]) 9 May 2013 (2013-05-09) * paragraphs [0004], [0038], [0052] - [0059]; claims 1-13; figures 1A,2,3A * | 1-6, 10-14 | INV. C07K17/08 C07K17/12 C12N11/08 C12N11/12 G01N33/544 |
| X | WO 2004/088316 A1 (COUNCIL SCIENT IND RES [IN]) 14 October 2004 (2004-10-14) * claims 1,8,18,21,23; examples 5-7, 10, 11, 16 * | 1-6, 10-14 | |
| X | DE 10 2005 011926 A1 (DEUTSCHES TEXTILFORSCHZENTRUM [DE]) 6 October 2005 (2005-10-06) * paragraph [0012]; claims 1-12; examples 1,2 * | 1-6, 10-14 | |
| X | WO 2005/083435 A1 (RUEHE JUERGEN [DE]; KLAPPROTH HOLGER [DE]) 9 September 2005 (2005-09-09) | 11-14 | |
| A | * page 7; claims 1-16; figure 1 * | 1-6,10 | |
| X | KAORI ABE ET AL: "Simultaneous Immunoassay Analysis of Plasma IL-6 and TNF-[alpha] on a Microchip", PLOS ONE, vol. 8, no. 1, 9 January 2013 (2013-01-09), page e53620, XP055167697, DOI: 10.1371/journal.pone.0053620 | 11-14 | TECHNICAL FIELDS SEARCHED (IPC) C07K C12N G01N |
| A | * abstract; figure 1 * * page 2 * | 1-6,10 | |
| X | US 2009/069199 A1 (BRANDENBURG ALBRECHT [DE]) 12 March 2009 (2009-03-12) | 11-14 | |
| A | * paragraphs [0003], [0008], [0059]; claims 1,6,7,24-29; figure 1 * | 1-6,10 | |
| | -/-- | | |

1 ~~The present search report has been drawn up for all claims~~

| Place of search | Date of completion of the search | Examiner |
|---|---|---|
| The Hague | 13 February 2015 | Schmidt-Yodlee, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 18 0423

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 562 961 B1 (SEEGER STEFAN [DE] ET AL) 13 May 2003 (2003-05-13) | 11-14 | |
| A | * column 1; claims 1-15 * | 1-6,10 | |
| | ----- | | |
| X | WO 01/81921 A2 (KIMBERLY CLARK CO [US]) 1 November 2001 (2001-11-01) | 11-14 | |
| A | * page 19; claims 1,10,19; examples 1-7 * | 1-6,10 | |
| | ----- | | |
| X | NADINE NOTTRODT ET AL: "Local ultraviolet laser irradiation for gradients on biocompatible polymer surfaces", JOURNAL OF BIOMEDICAL MATERIALS RESEARCH PART A, vol. 102, no. 4, 30 April 2014 (2014-04-30), pages 999-1007, XP055167992, ISSN: 1549-3296, DOI: 10.1002/jbm.a.34762 | 11-14 | |
| A | * abstract * <br> * page 1000; figures 1,6 * | 1-6,10 | |
| | ----- | | |
| | | | TECHNICAL FIELDS SEARCHED (IPC) |

1

~~The present search report has been drawn up for all claims~~

| Place of search | Date of completion of the search | Examiner |
|---|---|---|
| The Hague | 13 February 2015 | Schmidt-Yodlee, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 14 18 0423

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

6(completely); 1-5, 10-14(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 14 18 0423

The Search Division considers that the present European patent application does not comply with the requirements of unity of invention and relates to several inventions or groups of inventions, namely:

1. claims: 6(completely); 1-5, 10-14(partially)

   process for immobilizing biomolecules on a substantially non-porous carrier according to claim 1, wherein the carrier is composed of a naturally substantially non-porous material; carrier obtained by the process and its device, use and kit

---

2. claims: 7-9(completely); 1-5, 10-14(partially)

   process for immobilizing biomolecules on a substantially non-porous carrier according to claim 1, wherein the carrier is composed of a porous material that has been rendered substantially non-porous; carrier obtained by the process and its device, use and kit

---

## ANNEX TO THE EUROPEAN SEARCH REPORT ON EUROPEAN PATENT APPLICATION NO. EP 14 18 0423

This annex lists the patent family members relating to the patent documents cited in the above-mentioned European search report. The members are as contained in the European Patent Office EDP file on
The European Patent Office is in no way liable for these particulars which are merely given for the purpose of information.

13-02-2015

| Patent document cited in search report | Publication date | Patent family member(s) | Publication date |
|---|---|---|---|
| US 2013115558 A1 | 09-05-2013 | KR 20130050609 A | 16-05-2013 |
| | | US 2013115558 A1 | 09-05-2013 |
| WO 2004088316 A1 | 14-10-2004 | AT 378599 T | 15-11-2007 |
| | | AU 2003226633 A1 | 25-10-2004 |
| | | CN 1809752 A | 26-07-2006 |
| | | DE 60317571 T2 | 25-09-2008 |
| | | EP 1608972 A1 | 28-12-2005 |
| | | IL 171177 A | 29-12-2011 |
| | | WO 2004088316 A1 | 14-10-2004 |
| DE 102005011926 A1 | 06-10-2005 | NONE | |
| WO 2005083435 A1 | 09-09-2005 | AT 467126 T | 15-05-2010 |
| | | CA 2558187 A1 | 09-09-2005 |
| | | DE 102004010430 A1 | 22-09-2005 |
| | | EP 1721160 A1 | 15-11-2006 |
| | | JP 2007525681 A | 06-09-2007 |
| | | US 2008293592 A1 | 27-11-2008 |
| | | WO 2005083435 A1 | 09-09-2005 |
| US 2009069199 A1 | 12-03-2009 | DE 102007033124 A1 | 22-01-2009 |
| | | US 2009069199 A1 | 12-03-2009 |
| US 6562961 B1 | 13-05-2003 | AU 732821 B2 | 03-05-2001 |
| | | AU 9264698 A | 16-03-1999 |
| | | CN 1268142 A | 27-09-2000 |
| | | DE 19736736 A1 | 25-02-1999 |
| | | DK 1005495 T3 | 27-05-2002 |
| | | EP 1005495 A1 | 07-06-2000 |
| | | ES 2174489 T3 | 01-11-2002 |
| | | JP 2001514267 A | 11-09-2001 |
| | | US 6562961 B1 | 13-05-2003 |
| | | WO 9910383 A1 | 04-03-1999 |
| WO 0181921 A2 | 01-11-2001 | AT 375513 T | 15-10-2007 |
| | | AU 5152201 A | 07-11-2001 |
| | | AU 2001251522 B2 | 07-09-2006 |
| | | CA 2406511 A1 | 01-11-2001 |
| | | CN 1437708 A | 20-08-2003 |
| | | DE 60130879 T2 | 07-02-2008 |
| | | EP 1277056 A2 | 22-01-2003 |
| | | MX PA02009829 A | 27-03-2003 |
| | | TW I239399 B | 11-09-2005 |
| | | WO 0181921 A2 | 01-11-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 1161722
- FR 1260083


### Non-patent literature cited in the description


- **ABE et al.** *Anal. Bioanal. Chem.,* 2010, vol. 398, 885-893
- **ABE et al.** *Anal. Chem.,* 2008, vol. 80, 6928-6934
- **JARUJAMRUS et al.** *Analyst,* 2012, vol. 137, 2205-2210
- **KHREICH et al.** *Toxicon,* 2009, vol. 53, 551-559
- **JOHANSSON ; CAMPBELL.** *Surf. Interface Anal.,* 2004, vol. 36, 1018-1022
- **KHREICH et al.** *Anal. Biochem.,* 2008, vol. 377, 182-188
- **NGOM et al.** *J. Mater. Chem. B,* 2013, vol. 1, 3277-3286
- **ROY et al.** *Chem. Soc. Rev.,* 2009, vol. 38, 2046-2064
- **POSTHUMA-TRUMPIE et al.** *Anal. Bioanal. Chem.,* 2009, vol. 393, 569-582
- **NGOM et al.** *Anal. Bioanal. Chem.,* 2010, vol. 397, 1113-1135
- **FRIDLEY et al.** *MRS Bull.,* 2013, vol. 38, 326-330
- **NOH et al.** *Anal. Chem.,* 2010, vol. 82, 4181-4187
- **JOHANSSON.** Campbell, J. M. Reproducible XPS on biopolymers: cellulose studies. *Surf. Interface Anal.,* 2004, vol. 36, 1018-1022
- **MÄÄTTÄNEN, A. et al.** *Colloids Surfaces A Physicochem. Eng. Asp.,* 2010, vol. 367, 76-84